Europäisches Patentamt

European Patent Office

Office européen des brevets

(11) Publication number: **0 052 004**
A1

# EUROPEAN PATENT APPLICATION

(12)

(21) Application number: 81305311.3

(22) Date of filing: 09.11.81

(51) Int. Cl.³: **A 61 M 1/03**

(30) Priority: 12.11.80 US 206017

(43) Date of publication of application: 19.05.82
Bulletin 82/20

(84) Designated Contracting States: AT BE CH DE FR GB IT
LI LU NL SE

(71) Applicant: PURDUE RESEARCH FOUNDATION,
Graduate House East, West Lafayette
Indiana 47907 (US)

(72) Inventor: Ash, Stephen Richard, 2500, North County
Road, 400 East Lafayette Indiana 47905 (US)
Inventor: Hillberry, Benny Max, 246, Lincoln Street, West
Lafayette Indiana 47906 (US)
Inventor: Roberts, Randall Keith, 1358, Jefferson
Avenue, Salem Ohio 44460 (US)

(74) Representative: Hudson, Christopher Mark, Erl Wood
Manor, Windlesham Surrey GU20, 6PH (GB)

(54) **System and method for controlling and monitoring blood or biologic fluid flow.**

㊗ A system and method are disclosed for controlling blood flow or flow of biologic fluid in an artificial organ or extracorporeal blood circuit. Selected parameters relating to blood flow are sensed and used to automatically optimize blood flow while assuring proper and safe operation. Utilized in conjunction with a reciprocating dialyzer apparatus (21), the system controls blood flow into and out of the dialyzer apparatus by controlling pressure on a slurry around the dialyzer membrane package. Slurry pressure is controlled by an actuator (16), such as a bellows pump, the pumping action of which is controlled by an electric motor (21), which motor, in turn, is electrically controlled by a control unit (29) which receives sensed indicators of blood flow, pressure and/or motor current. Blood contamination is also monitored for assuring safe operation of the dialyzer apparatus.

ACTORUM AG

## SYSTEM AND METHOD FOR CONTROLLING AND
## MONITORING BLOOD OR BIOLOGIC FLUID FLOW

This invention relates to a blood or other biologic fluid control system and method and, more particularly, relates to a system and method for controlling fluid flow in a dialyzer apparatus, particularly of the reciprocating type.

As is well known, the kidneys are organs in the body that, among other things, filter and excrete waste products from the blood, as well as removing excess water via ultrafiltration. If the kidneys should fail to function properly, and not adequately treat the blood to remove impurities therefrom, a condition, known as uremia, results which is characterized by increased levels of waste substances in the blood. When uremia results, a kidney transplant can be performed to remove or alleviate the problem, or, as is more often the case, the uremia is controlled by artificial means.

The most common artificial treatment of kidney failure is hemodialysis which involves the diffusion of metabolic waste products across semipermeable membranes, as well as removal of water by filtration across the membrane. In the design of an artificial kidney, an attempt is made to maximize the rate at which the blood is treated by the artificial kidney while also ultrafiltrating the appropriate amount of fluid during each treatment.

A measure of efficiency of dialysis is clearance, which is defined as the amount of blood that is totally cleared of solute therein in one minute. The higher the clearance, the lower the average blood concentration will be with respect to toxic substances.

In order to maximize clearance, it is necessary, among other things, to maximize blood flow. Accordingly, a good design for artificial kidneys optimizes the blood flow both into and out of the artificial kidney. But this has proved to be a difficult task due, at least in part, to the necessity of removing the blood from highly compliant vessels, such as veins.

While attempting to optimize blood flow, care must also be taken to assure pressure control for both blood and dialysate. The ultrafiltration rate is approximately proportional to the pressure difference across the membranes, and has been found to be approximately proportional to the pressure gradient. Control of the pressure gradient has, however, also been found to be difficult when a flow change of blood occurs.

Among dialyzers now known, three types are commonly utilized, one of which is known as a coil dialyzer, a second of which is known as a hollow fiber dialyzer, and the third of which is known as a parallel plate dialyzer. All of these devices are of the so-called "single pass" type of dialyzer apparatus in which the blood flows in one direction through a conduit into the dialyzer apparatus and then is discharged from the dialyzer apparatus by flow of the treated blood in one direction through another conduit. While "single pass" dialyzers have been used with some success, problems have nevertheless been encountered.

A solution to some of these problems is provided by an artificial kidney, called a sorbent suspension, reciprocating hemodialyzer (SSRD) which is described and claimed in U.S. Patent No. 4,071,444. This type of dialyzer has a single blood access via a single, large bore, intravenous catheter. Blood is pumped by the membranes in response to pressure changes on the dialysate side, rather than by a blood pump. The dialyzer is lightweight and portable so as to be suitable for use for relatively long dialysis periods, uses a sorbent suspension (such as charcoal, urease, and zeolite) to maintain a steep transmembrane concentration gradient (without need for a large dialysate volume), and provides for expandable and collapsible membrane packages.

The dialyzer apparatus shown in U.S. Patent No. 4,071,444, when used as a portable, self-contained unit, can, in contrast to traditional equipment, free the patient from the confines of a chair or a bed in a hospital treatment center. Such a wearable device is simple enough to allow for patient self-dialysis, allows dialysis on a very reasonable daily basis which correlates much closer to normal kidney function, and conceptually is thus better for the patient. The added mobility afforded also adds to patient acceptance of treatment sessions and can allow longer sessions with improved health.

To achieve the desired ends, however, such a dialyzer apparatus must be capable of cycling the blood in a manner which maximizes clearance and controls the rate of ultrafiltration.  With respect to a reciprocating dialyzer (i.e., a dialyzer in which the blood is introduced into and removed from the blood treatment cell through the same conduit), use of a venous catheter is made possible, but with the use of central, low pressure veins, the problem of venous collapse is accentuated.  With the SSRD dialyzer apparatus, the blood can be contaminated by membrane failure, for example, which could cause slurry to be introduced into the blood.  Such contamination is to be avoided, and if such contamination should occur, operation of a dialyzer apparatus should be immediately terminated.

Similar problems of movement occur with other biologic fluids, such as peritoneal fluid.  Peritoneal dialysis is performed by placing a sterile, balanced salt solution in the peritoneal cavity, through a single access device, allowing time for diffusion of toxic substances into the fluid and removing the fluid. The movement of this fluid is often accompanied by obstruction of the catheter, and the flow rate must be adjusted to the maximum attainable.  In addition, particulate contamination of the fluid must be assiduously avoided.

This invention provides a system and method for controlling flow of blood or other biologic fluid and assuring proper and safe operation with the system and method being particularly useful for controlling blood or other fluid flow in an extracorporeal circuit, and particularly in the reciprocating dialyzer apparatus. Selected parameters related to blood or other fluid flow are sensed and utilized to both optimize blood or other fluid flow and assure proper and safe operation.

Specifically, this invention provides a system for controlling biologic fluid flow, which comprises fluid flow effecting means for causing fluid flow in a predetermined path; first monitoring means for monitoring fluid flow in the predetermined path and providing an output indicative of at least one preselected parameter thereof; second monitoring means connected with the fluid flow effecting means and providing an output indicative of at least one preselected parameter thereof; and processing means connected with the first and second monitoring means and the fluid flow effecting means to receive the outputs from the first and second monitoring means, and, at least partially responsive thereto, providing an output to the fluid flow effecting means to automatically control fluid flow in the predetermined path.

Also, this invention provides a control system and method for controlling blood or other biologic fluid flow.

Additionally, this invention provides a control system and method for controlling blood or other biologic fluid flow in a dialyzer apparatus and particularly in a reciprocating dialyzer apparatus.

Finally, this invention provides a control system and method for controlling blood or other biologic fluid flow in a dialyzer by sensing selected parameters of the blood and utilizing the same to optimize flow and assure proper and safe operation.

It will become apparent to one skilled in the art as the description proceeds, that this invention resides in the novel construction, combination, arrangement of parts and method substantially as hereinafter described and more particularly defined by the appended claims, it being understood that such changes in the precise embodiments of the herein disclosed invention are meant to be included as come within the scope of the claims.

The accompanying drawings illustrate complete embodiments of the invention according to the best mode so far devised for the practical application of the principles thereof, and in which:

FIGURE 1 is a block diagram of the control system shown in conjunction with a reciprocating dialyzer apparatus;

FIGURE 2 is a representative equivalent diagram of the blood treatment cell shown in block form in FIGURE 1;

FIGURE 3 is a plot of pressure versus volume for a blood treatment cell to establish a typical membrane compliance curve;

FIGURE 4 is an electrical equivalent schematic circuit for a blood treatment cell;

FIGURE 5 is a plot illustrating theoretical response of a blood treatment cell to a step in slurry flow;

FIGURE 6 is a plot illustrating theoretical response of a blood treatment cell to a step in case pressure;

FIGURE 7 is an expanded block diagram of the control system shown in conjunction with a reciprocal dialyzer apparatus;

FIGURE 8 is a flow chart of a program for the control system as shown in FIGURE 7;

FIGURE 9 is a schematic diagram of a mechanical and electrical system model of a pumping system of a reciprocal dialyzer apparatus;

FIGURE 10 is a plot of motor current versus bellows displacement for computing equivalent case pressure;

FIGURE 11 is a flow chart of a program for a control system using motor current as case pressure equivalents;

FIGURE 12 is an illustration of venous catheter location points in a test animal;

FIGURE 13 are plots of dialyzer apparatus response for catheter locations as shown in FIGURE 12;

FIGURE 14 are plots of motor current and pressure during operation of a pressure type controller;

FIGURE 15 are plots of in vitro motor current type controller response;

FIGURE 16 is a front panel view of an alternate embodiment of a controller unit that operates with a fixed program and has operator adjustable parameters and operational controls;

FIGURE 17 (A, B, and C) illustrate circuitry utilized in controller unit shown in FIGURE 16;

FIGURE 18 is a schematic of an alternate embodiment of a pumping unit usable as a part of this invention;

FIGURE 19 (A) is an illustration in block form of a contamination detection device that can be utilized in the invention to detect blood contaminants;

FIGURE 19 (B) and 19 (C) are Doppler spectrum (power versus frequency) of continuous and pulsed Doppler with respect to pure blood and blood having different values of contaminants therein; and

FIGURE 19 (D) is a graph of data obtained from Doppler spectra as a function of carbon concentrations.

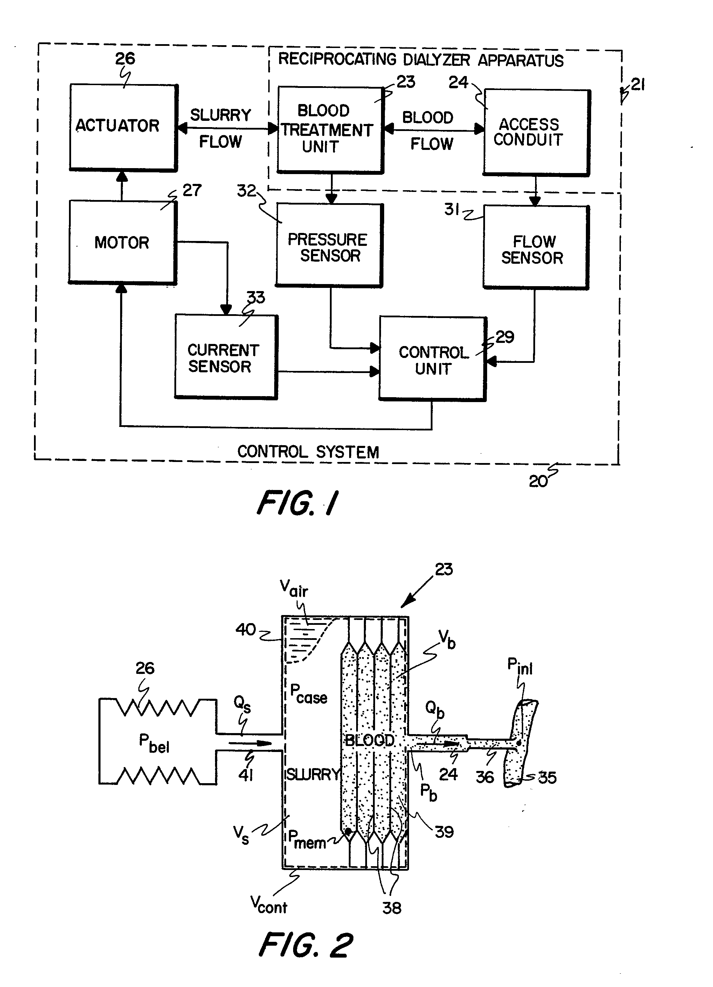

The control system 20 of this invention is shown in conjunction with a reciprocating dialyzer apparatus 21. As shown, apparatus 21 includes a blood (or other biologic fluid) treatment unit, or cell, 23 and an access conduit 24 through which blood (or other biologic fluid) is introduced into and withdrawn from treatment unit 23. Slurry flow into and out of treatment unit 23 is effected by actuator 26 which is controlled by motor 27. Motor 27 is, in turn, controlled by control unit 29 which receives sensed parameters relating to blood flow, which as shown in FIGURE 1, include a flow sensor 31, a pressure sensor 32 and/or a current sensor 33.

The use of single access conduit for dialysis represents an advantage to a patient. However, when used with a central venous catheter, an adequate control system is required to prevent and/or correct venous collapse.

A venous catheter becomes occluded when the venous wall collapses around the catheter tip. This phenomenon is related to the rate of blood withdrawal,

0052004

and the upper flow limit of blood removal via venous catherization occurs when the flow exceeds the local supply of blood near the catheter tip. When this occurs, there is a reduction in the pressure around the catheter tip, and collapse of the venous wall occurs around the catheter, at which time the resistance of the catheter approaches infinity. This phenomenon is well known in medicine as a limitation to blood withdrawal.

While the compliance of the venous system during blood withdrawal has not been thoroughly studied, it is felt probable that the collapse phenomenon is related to local venous compliance, to internal pressure in the vein, and to the distance between the tip and the venous wall. Veins have been found to be far more compliant than arteries, and are highly compliant during inflow of fluid (more studies of venous compliance have been performed during expansion of the venous system than during drainage, but it has been shown that during drainage, collapse occurs at any pressure less than atmospheric). Testing, both in vitro and in vivo, with eight and fourteen guage venous catheters, has demonstrated that flow resistance increases as fluid withdrawal rates increase. With a venous catheter system, it has been found that:

1. The inlet resistance to the catheter is a relatively constant resistance, and may be calculated utilizing only a few basic assumptions;

2. The resistance to flow from a venous catheter during blood removal is metastable, that is, obstruction to the flow may occur suddenly, at varying pressures and flows, and may be the result of small changes in catheter position with respect to the venous wall or to changes in local venous geometry (deformation);

3.  The total collapse phenomena of the vein may be partially duplicated in vitro by mechanical vein models having a two-tube system with constant pressure gradient for flow through the model (inlet resistance increases with increasing flow, and a collapse phenomenon at variable flow rate is present, but oscillations occur during high resistance periods which do not occur during in vivo tests, and for this reason, the model can be used only if the pressure signal with respect to the blood tubing is not used as an indication of vein collapse);

4.  The local compliance of a vein is not the same as the overall compliance of the vein, and compliance measured on outflow of fluid is markedly different from that measured on inflow;

5.  Once a vein collapses around a catheter, the catheter stays obstructed until flow is reversed;

6.  The withdrawal flow rates obtainable from a vein may be less than the total maximal flow in that vein, and a venous catheter may therefore deliver only a fraction of the actual flow in the vein;

7.  Small changes in tip angle and tip location can greatly affect the maximal blood flow obtainable before collapse;

8.  One-way flows over 300 ml/min, at maximum pressures of 300 mm of Hg, are possible for a venous catheter of about eight guage size; and

9.  Large flow rates are not stable, with obstructions occurring suddenly with no pressure or flow changes which precede this phenomenon that allow the prediction of impending collapse.

From the foregoing, it can be seen that a successful system and method for blood flow control must be adaptive, and factor in previously successful and unsuccessful flow rates, in order to optimize blood withdrawal.  In addition, such a control system and method must provide a "reverse" mode to reopen obstructed cannalae since obstruction cannot be fully predicted and avoided.

A dialyzer apparatus, functioning as an artificial kidney, is shown and described in U.S. Patent No. 4,071,444 issued January 31, 1978, entitled "Portable Chemical Reactor For Use As An Artificial Kidney" and assigned to Purdue Research Foundation, which patent is hereby included by reference.  The apparatus shown is a sorbent suspension, reciprocating hemodialyzer (SSRD) wherein blood is drawn into a blood treatment chamber, treated, and then expelled from the treatment chamber through the same conduit as used to draw the blood into the chamber.

As shown in U.S. Patent No. 4,071,444, blood flows into the dialyzer apparatus through a center hole and is distributed by center gaskets into circular membrane packages forming a blood chamber between the membranes.  Slurry is introduced at the periphery of dialysate chambers formed at each side of the membrane package.  Such a dialyzer may also be used for removal, treatment, and return of peritoneal dialysis fluid, so long as the fluid contains a small number of cellular contaminants for ultrasonic scattering.

With single pass dialyzers, the membrane surface area to blood volume ratio is maximized by limiting the blood channels formed by the membrane packages to about 0.5 mm to 1.0 mm in width. The typical diameter of the blood treatment cell as utilized in this invention is preferably about 120 mm and a 70 ml fill volume dialyzer apparatus contains about 15 membrane packages.

In general, blood treatment cell 23 can be represented by the diagram of FIGURE 2 which illustrates the blood treatment cell 23 in communication with a vein 35 of a user by means of conduit 24 which terminates in venous catheter 36. Cell 23 includes a plurality of pairs of membranes 38 which each pair establishing a blood chamber 39 within sealed case 40. Blood is introduced into and taken from blood chambers 39 through access conduit 25 and catheter 36. Slurry is introduced into and withdrawn from case 40 through conduit 41 in fluid communication with actuator 26, which is shown in FIGURE 2 to be a bellows pump which has the slurry stored therein (so that the bellows pump provides a reservoir of slurry and also serves as a volumetric fluid pump).

A volume of air is illustrated within case 40 to show its effects, but such air would never intentionally be left in the case and is removed therefrom prior to operation.

A volume control is defined so that

$$V_{cont} = V_s + V_b + V_{air} \qquad (1)$$

Assuming $V_{cont}$ to be a constant, differentiation of equation (1) with respect to time yields

$$\dot{V}_{air} + \dot{V}_b + \dot{V}_s = o \qquad (2)$$

For one direction of flow (as shown in FIGURE 2),

$$\dot{V}_b = -Q_b \qquad (3)$$

$$\dot{V}_s = Q_s \qquad (4)$$

Substituting equations (3) and (4) into equation (2),

$$Q_b(t) = Q_s(t) + \ddot{V}_{air}(t) \qquad (5)$$

where $\dot{V}_{air}(t)$ = rate of increase in $V_{air}$

If $V_{air}(t)$ can be expressed in terms of known variables, then it can be differentiated with respect to time and the value of $Q_b(t)$ and be found knowing $Q_s(t)$.

Assuming an initial mass of air at a known condition is given, then

$$V_o = (m_o R T_o)/P_o \qquad (6)$$

where $m_o$ = mass of air

$R$ = universal gas constant

$T_o$ = temperature when $V_o$ recorded

$P_o$ = pressure when $V_o$ recorded

Then for an isothermal expansion, the volume of air at time t is given as

$$V_{air}(t) = P_o V_o / P_{case}(t) \qquad (7)$$

Differentiating with respect to time yields

$$\dot{V}_{air}(t) = -[K_a \dot{P}_{case}(t)]/(P_{case})^2 \quad (8)$$

where $K_a = P_o V_o$

Thus, if the case pressure can be related to the flows, $Q_s$ and $Q_b$, the $\dot{V}_{air}(t)$ can be eliminated from equation (5) and an explicit solution for $Q_b$ in terms of $Q_s$ can be determined.

The blood side pressure, $P_b$, can be related to blood flow by the relationship

$$P_b = P_v + (R_{cath} + R_{inl})Q_b \qquad (9)$$

If the pumping system utilizes a bellows, as shown in FIGURE 2, case pressure is generated through a tube (conduit 41) connecting cell 23 to bellows 26, and the tube has a finite fluid resistance, $R_t$, so that:

$$P_{bel} = P_{case} + R_t Q_s$$

where $P_{bel}$ = average pressure in bellows     (10) Due to the limiting compliance of the cell membranes and the spring effect of the membrane elasticity, the pressures on either side of the membranes are related by

$$P_{case} = P_{mem} - K_{mem}(V_b)$$

where $P_{mem}$ = pressure on blood side in membrane package

$K_{mem}$ = transmembrane pressure gradient

This transmembrane pressure gradient is caused by the limiting compliance of the membrane packages. That is, there is a finite limit to the amount of blood that can be drawn into a package.

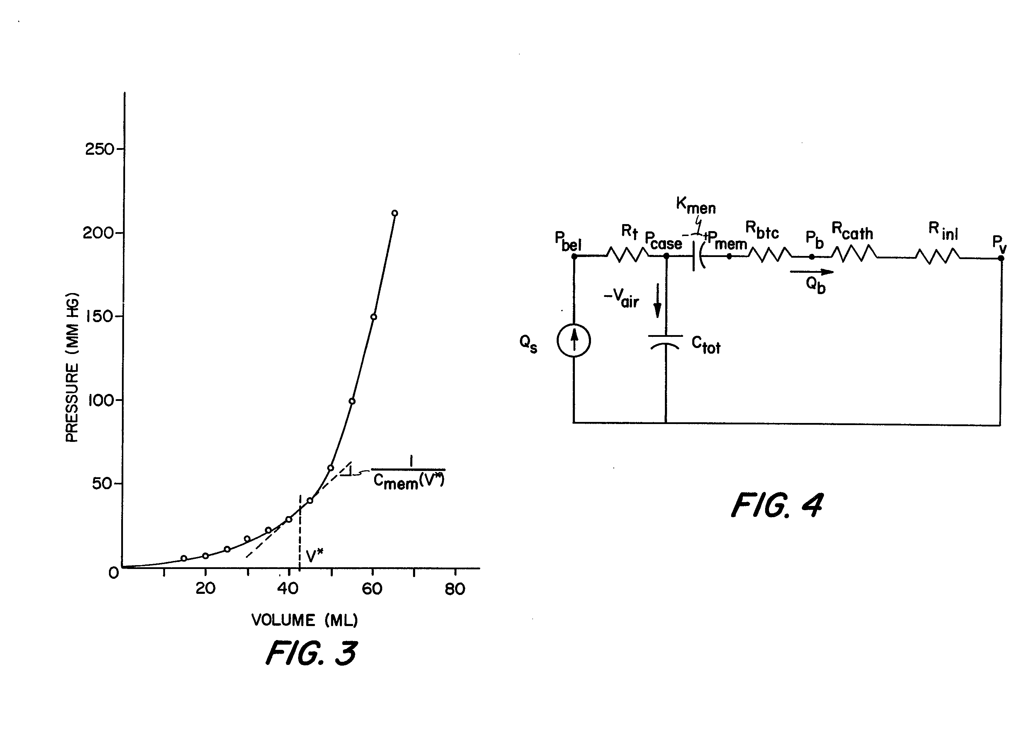

In FIGURE 3, data for pressure versus volume for the blood treatment cell are plotted. To obtain test data, a syringe was hooked up to the blood inlet port of the cell and volumes of fluid were injected into the cell. For the plot, a pressure measurement, at the tubing outlet, was recorded along with the amount of volume infused. The membrane compliance, $C_{mem}$, is defined as the amount of volume change per given pressure variations. Thus, $C_{mem}$, for a given blood volume, is the inverse of the tangent to the pressure-volume curve. For a cell relating to the results plotted in FIGURE 3, the membrane packages cannot expand to accommodate more than about 70 cc of fluid, and, as such, $C_{mem}$ goes to zero at this volume. The pressure drop due to the blood gaskets and membrane channels, defined as $R_{btc}$, causes pressure drops given by:

$$P_b = P_{mem} - R_{btc}Q_b \qquad (12)$$

Combining equations (8) through (12) with equation (5) yields:

$$(\tau_p D + 1)P_{case}(t) = R_{tot}Q_s(t) + P_v - K_{mem}(V_b) \qquad (13)$$

where D = time derivative (d/dt)

$$\tau_p = \text{system time constant} = C_{tot}R_{tot}$$

$$R_{tot} = \text{total flow resistance} = R_{cath}+R_{inl}+R_{btc}$$

$$C_{tot} = \text{system compliance} = C_{case}+K_a/(P_{case})^2$$

The total system compliance is the sum of two factors, the compliance of the cell itself and the compressibility of the trapped air in the case. The term $C_{case}$ is termed the case compliance and represents the change in $V_{cont}$ caused by changes in case pressure. Typically, this compliance is small for a cell case made of 6 mm molded acrylic. Equation (13) shows that square waves of case pressure cannot be generated by finite values of $Q_s$. A step change in slurry flow causes a first order response in case pressure. The time constant, and therefore the speed of response, is related to the system compliance and resistance. Thus, if the cell has air leaks, the ability to create rapid changes in case pressure is reduced. The derived equation also points out the problems with a constant volume pump when run without a pressure monitor. The value of $K_{mem}$ goes to infinity for limiting blood volumes, thus equation (13) indicates that after some time delay, the case pressure would also reach infinity in steady state. When a constant volume is used, it would be quite easy to generate large values of case pressure at the end of each cycle.

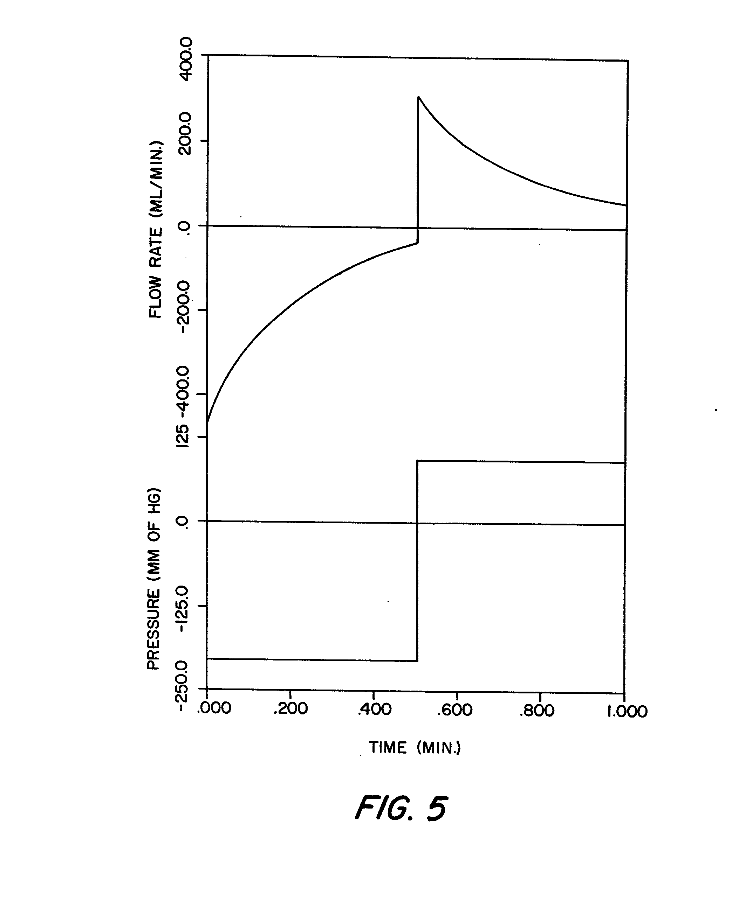

The previous equations were simulated on a computer, and all the values of the given variables were input to the program. The set of equations presented above are represented by the circuit shown in FIGURE 4. A simulation was run for two different cases, volumetric slurry pumping and pressure-vacuum air pumping. The values for the flow resistances were:

$$R_{inl} = 0.001Q_b;$$
$$R_{cath} = 0.5; \text{ and}$$
$$R_{btc} = 0.1 \text{ ml/min/mm Hg}.$$

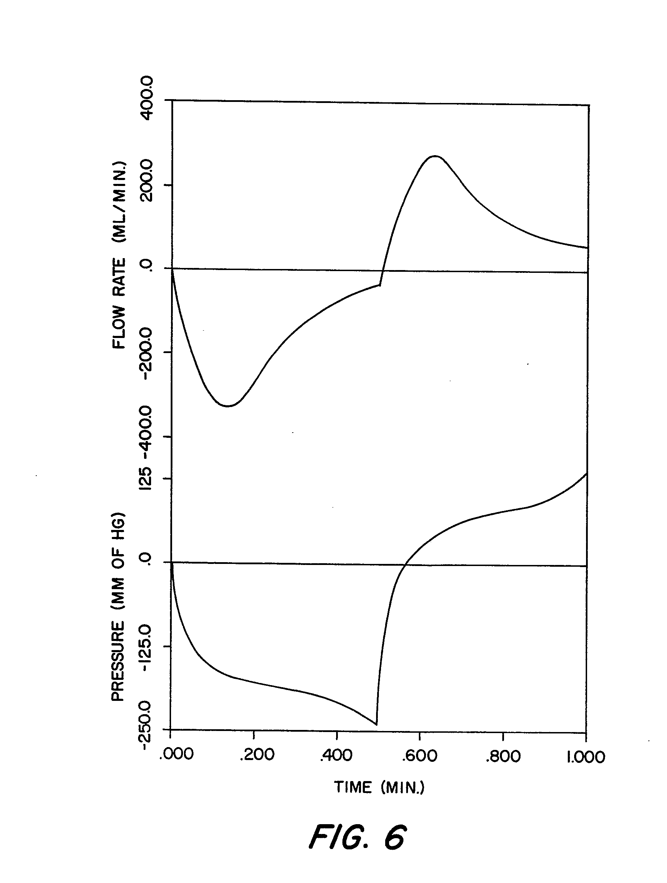

The initial volume of air in the case was set to 10 cc and the membrane compliance curve of FIGURE 3 was used. The theoretical response of the blood treatment cell are shown in FIGURES 5 and 6 to a step in slurry flow (FIGURE 5) and to a step in case pressure (FIGURE 6). It can be readily seen that the step in case pressure produces rapid variations in blood flows.

From the foregoing, the following conclusions can be reached:

1. Using a system that injects fluid into the case to vary the case pressure, a square wave of case pressure cannot be realized;

2. A pressure pump system will theoretically cause unbounded blood flow rates, i.e., when the case pressure exhibits a step change, the instantaneous blood flow rates must approach infinity since the term $\tau_p DP_{case}$ is an impulse (this abrupt change in the flow would, in general, cause the access to occlude more readily than a continuous variation in case pressure);

3. In general, the response of the blood treatment cell has a time delay that is determined by the compliance and resistance of the cell (thus, the more air in the case, the slower the system responds); and

4.  The case pressure should constantly be monitored since this pressure can increase rapidly at the end of each cycle from a volumetric slow system (the volume flow rate pump system will not work, even if the limits of bellows excursion are set, because the ultrafiltration volume causes $V_s$ to increase, and thus there will be a shift in the baseline pressure and extreme positive pressures will be generated).

Control system 20 has the following characteristics:

1.  Actuator 26 (pump) attempts, at all times, to pump the blood in a manner that maximizes the dialyzer's blood treatment rate (it has been found that, in general, to increase the clearance, the blood must be introduced into and withdrawn from the cell as fast as possible, i.e., the blood volume in the membrane packages should approximate a square wave with time, and the pumping system therefore at all times strives to maximize the blood inflow and outflow rates and avoid prolonged periods with the blood chamber formed by the membranes empty of blood);

2.  Control is provided with respect to the amount of ultrafiltration (this rate has been shown to be approximately proportional to the transmembrane pressure gradient which is assumed to be a constant for most single pass dialyzers so that the total volume ultrafiltered during a treatment session can easily be estimated, but for a reciprocal dialyzer, termed $K_{mem}$ in the derivations, this rate is not constant and depends on the volume of blood in the membrane packages which ranges from zero with no blood volume to a maximum for filled packages, and, therefore, the pump has a delay period where the blood is held in the membrane packages so ultrafiltration is maximized for this short period in the overall cycle);

3. Limits have been established for positive and negative case pressures to prevent membrane rupture (for this reason, a pressure monitor is utilized because of the change of over pressurization, and such a signal is sampled at a sufficiently high rate to allow for pressure limitation);

4. Blockage of blood flow, due to venous collapse, tubing occlusions, or blood clotting in the cell, is identified and, if possible, prevented (the blood flow rate is constantly monitored in an attempt to identify blood line blockage with a zero flow condition during inflow into the dialyzer being presumed to be caused by venous collapse, at which time the pump should reverse the flow in an attempt to reopen the access);

5. The system is adaptable to varying cell designs which have different fill volumes, flow resistances, and membrane compliances (for this reason the user inputs to the pumping system are variable according to the cell design, for example, an input of inflow cycle time would be inappropriate since the amount of blood fill volume could vary from cell to cell, and a better input is the time to hold the blood in the filled membrane packages); and

6. The system is able to detect air bubbles or slurry particles in the blood and terminate operation when sensed (the safety monitors for blood contamination use an ultrasound flowmeter Doppler system, and such a system has been shown to be capable of detecting a charcoal dilution of 1 in 50,000 in blood).

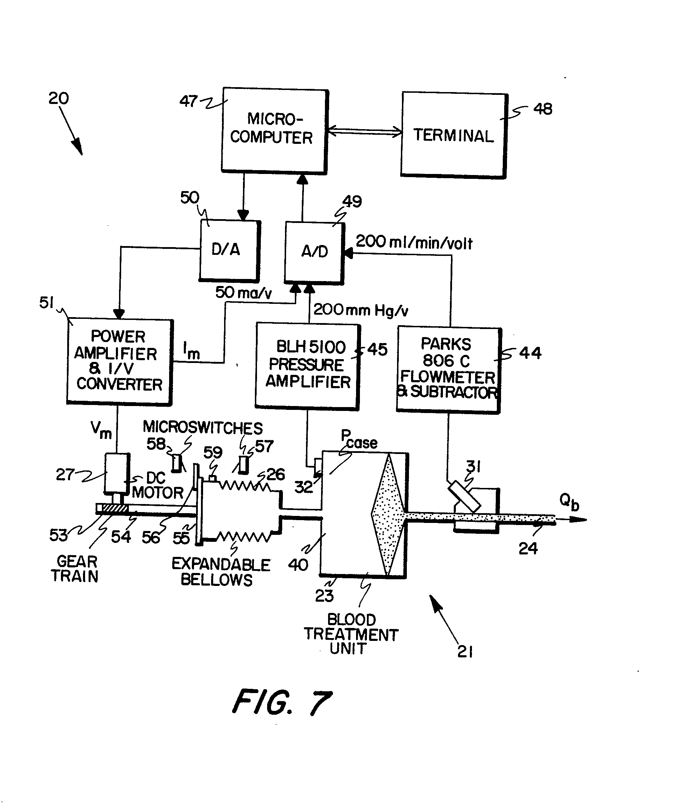

Control system 20 is shown in greater detail in FIGURE 7 in conjunction with reciprocal dialyzer apparatus 21 having blood treatment unit 23 and access conduit 24. As shown, flow sensor 31 is a transducer positioned at conduit 24 with the output being coupled to a Parks 806C flowmeter and subtractor unit 44; and pressure sensor 32 is positioned at case 40 of cell 23 with output being amplified by a BLH 5100 pressure amplifier 45. Control unit 29 of FIGURE 1 is shown in FIGURE 7 to include a microcomputer 47 connected with terminal 48. Microcomputer 47 is connected with flowmeter 44 and pressure amplifier 45 through analog-to-digital converter 49, and with DC motor 27 through analog-to-digital converter 50 and power amplifier 51. Amplifier 51 can include a current-to-voltage converter to provide a motor current output to the microcomputer 47 through analog-to-digital converter 49. This current output (Im) from unit 51 may be used in lieu of the pressure transducer output as brought out more fully hereinafter.

As also shown in FIGURE 7, DC motor 27 drives bellows 26 through a conventional gear train 53 which causes longitudinal displacement of shaft 54 attached to back plate 55 of bellows 26. As can be appreciated from FIGURE 7, displacement of shaft 54 causes displacement of back plate 55 which controls expansion and contraction of expandable bellows 26 in conventional fashion. As also can be appreciated from FIGURE 7, back plate 55 has an ear 56 thereon to contact microswitches 57 and 58 which were positioned to establish maximum and minimum limits of travel of the bellows, again in conventional fashion.

There are numerous units that could function as the actuator, or pump, for the blood treatment cell. An air pump system (not shown) could be utilized, for example, having a small compressor and vacuum pump along with sequenced valving to create positive and negative case pressure. The preferable type of unit, however, creates a variable case pressure by pumping slurry into or out of the cell with a simple expandable chamber (bellows) 26. This type of actuator has several advantages, among which are that it is generally a simpler hardware configuration, requires less power to operate, and offers the greater chance for portability.

In a working embodiment of the invention, bellows 26 was approximately 90 mm in diameter and expanded from 125 mm in length. The drive for pump 26 was provided by a small permanent magnet DC electric motor 27 (an Escap Model 22C11-216 motor, Portescap U.S., New York, New York), and gear train 53 was a 64:1 gearhead driving a 30:1 worm gear reducer. The output shaft turned a 0.5" diameter pinion gear with a rack mounted to the bellows. The translation of the bellows caused the slurry to be pumped into the rigid case 40, thus varying the case pressure. Bellows 26 also had a removable cap 59 so that it could be easily filled and cleaned.

Utilizing bellows 26 as a volumetric pump, case pressures are rapidly built up at the extremes of the bellows cycle. Venous collapse can occur, however, unless monitors are used to detect flow conditions. For this reason, closed-loop digital control unit 20 is provided with inputs indicative of case pressure and blood flow rate, as indicated in FIGURE 7. The case pressure signal feedback (sensed by pressure sensor 32) allows the control unit to determine when to reverse the cycle and also identify zero flow conditions. The blood side pressure signal $P_b$ (similar to $P_m$ in the flow study) provides redundant information.

Pressure transducer 32 (which in a working embodiment, was a Model RP 1500, Physiograph, Narco Systems, Houston, Texas) and amplifier 45 (which in a working embodiment, was a BLH 5100 Strain Gage Signal Conditioner) provides a sensitivity of 1.0 volt/200 mm Hg. The transducer is housed in a threaded plug that can be attached to a hole in case 40 of the blood treatment unit 23.

Measurement of blood flow is made by an ultrasound Doppler flowmeter 44 (which, in a working embodiment, was a Parks 806C - 10 MHz Directional Doppler, Beaverton, Oregon). This system operates based on Doppler principles, that is, a frequency shift caused by a velocity of the reflected medium. A continuous 10 MHz frequency is emitted by a probe, or transducer, 31, and the reflected sound wave is compared to the emitted wave by a phase detector to determine the freqency shift. An increase in the freqency indicates there is a flow toward the probe while a decrease indicates flow away from the probe. The flowmeter has separate outputs for both flow toward and away from the probe, and a voltage subtractor provides a bidirectional indication of the flow to the control system. The final flow indicator has a sensitivity of 1.0/200 ml/min outflow.

Control unit 47 is a microcomputer (which, in a working embodiment, was a Cromemco Z-2D, Mountain View, California) that at all times reads in sensed variables and adjusts the motor voltage accordingly. The analog signals are converted to 8 bit patterns by analog-to-digital (A/D) converter 49. The correct motor voltage is determined by the microcomputer program and outputted in the form of a 8 bit pattern which is converted to a voltage by digital-to-analog (A/D) converter 50. The computer used in a working embodiment had 64K memory, a 7 channel 8 bit A/D and D/A board, and a 5" magnetic disk drive. A terminal 48 (which in a working embodiment was a Lear Seigler ADM, Anaheim, California) is connected to microcomputer 47 to allow for user input, data display, and program development.

The output from D/A converter 50 is a low power signal, so operational amplifier circuit 51 was utilized having a variable voltage gain with an output of approximately two watts of power. The gain is arbitrarily set at 3.0 when using a D/A converter having a range of ± 2.50 volts with a recommended maximum motor voltage of ± 7.50.

The current-to-voltage converter (I-V converter) of amplifier unit 51 is a circuit that senses the current drawn by the motor and converts this current to a proportional measurable voltage by amplifying the voltage drop across a small series resistor in the drive circuit. This proportional voltage may be utilized for monitoring purposes, and may be used in lieu of the output of pressure transducer 32, as brought out more fully hereinafter. The sensitivity of the circuit is 1.0 Volt/50 ma.

The function of the control unit is to determine what the motor voltage should be at any time given the current and desired values of case pressure and flow rate. The program to achieve this end is written in the programming language BASIC.

The cycle for the reciprocal dialyzer is divided into three stages: inflow, delay, and outflow. Inflow is that phase in the overall cycle when blood is drawn into the cell, outflow is when blood is pumped back to the patient, and delay is a period that occurs when a user specified negative pressure is reached in the cell and is thereafter held for a user specified time. The adjustment of the negative pressure and time dictates the ultrafiltration volume.

During inflow, the controller maximizes the withdrawal rate, identifies and prevents venous collapse, and limits the negative pressure in the case. An increase in motor voltage causes an increase in blood flow rate, so to maximize the withdrawal rate the magnitude of the negative motor voltage is maximized.

The identification and prevention of vein collapse is accomplished by monitoring the blood flow rate. If the vein collapses around the catheter inlet, then the flow rate, $Q_b$, goes to zero regardless of the motor voltage, $V_m$. To identify this phenomena, the blood flow rate is compared to a small value, say 20 ml/min. If the sensed rate is lower, then the vein has collapsed around the catheter tip (or some obstruction has occurred in the tubing). This situation might be prevented by reducing the value of flow, $Q_b$, so that there is less tendency to collapse the vein. Also, once the vein has collapsed, back pressure must be reduced, or even reversed, to reopen the collapsed vein.

The motor voltage is incremented if there is sufficient flow so as to maximize the withdrawal rate. If the flow drops below a small value, then the system pumps a small amount of blood back until the case pressure is about 20 mm Hg. At that time, inflow is again commenced, but with a lower withdrawal rate in an attempt to avoid a repeat of vein collapse (which occurred at the higher withdrawal rate). The value of case pressure is checked regularly to insure that the limit of negative pressure is not exceeded. If exceeded, then the next phase of the cycle (the delay phase), is entered.

The delay stage holds the case pressure at a user specified pressure for a user specified time. It is during this part of the cycle that the majority of ultrafiltration occurs. In single pass dialyzers, ultrafiltration is controlled by adjusting the transmembrane pressure. This gradient is maintained at a constant during the entire session, thus an expected value of ultrafiltrate can easily be calculated. Charts of these relationships allow the user to pick the pressure and time of dialysis desired.

With the reciprocal dialyzer, however, the value of transmembrane pressure is not a constant throughout the cycle. This transmembrane pressure depends on the fill volume of the cell, spring constants of the membrane supports, and case pressure. It is the purpose of the delay phase to ultrafilter a desired amount of water and to allow diffusion of toxic chemicals across the membrane. When $P_{case}$ is held constant at $P_{neg}$, the transmembrane pressure, in steady state, becomes $K_{mem}(V_{max})$. Thus, the amount of water filtered during the delay time can theoretically be calculated for a specific cell design. When such a cell design is finalized, then similar graphs can be made that tell the user what value of $P_{neg}$ and delay time are needed.

The process of holding $P_{case}$ at $P_{neg}$ is a classic regulator problem. For the system of this invention, a digital feedback control unit was used because of the need for an adaptive system. The system plant is still analog, so the controlled variable is a continuous time varying function. Also, for the system of this invention, a proportional controller was used because of the relatively slow response. The difference between the desired and actual value creates an error signal that is fed directly into the system plant. Specifically, the equation used to determine the motor voltage output during the delay stage is:

$$V_m = 3K_g(P_{neg} - P_{case}) \qquad (14)$$

where $K_g$ = loop gain

$P_{case}$ = measured case pressure

$P_{neg}$ = user specified pressure .

The factor of 3 is the built in gain of the amplifier as the range of the D/A converted is $\pm$ 2.50 volts and the maximum voltage limit for the motor are $\pm$ 7.50 volts. The overall system will drive the case pressure to the value desired, $P_{neg}$. To illustrate this, suppose $P_{case} > P_{neg}$, then $V_m < 0$, so the motor will expand the bellows, thus decreasing $P_{case}$. If $P_{case} < P_{neg}$ the bellows will be compressed until $P_{case} = P_{neg}$. At this time the motor will stop, but will constantly oppose any force that tries to change the pressure. The digital feedback also must restrict the value of $V_m$ to be in the range of $\pm$ 7.50 volts because of motor limitations. The value of $K_g$ effects the speed of response of the system and was experimentally set at 1 v/50 mm Hg.

At the end of the delay stage, the control unit causes return of the treated blood back to the patient in minimal time while preventing overpressurization of the case. The control unit was designed using the same feedback system as during the delay stage. The user inputs two values, $P_{pos}$, the desired positive pressure, and $Q_{min}$, the lower limit for the outflow rate. The motor voltage is given by

$$V_m = 3K_g(P_{pos} - P_{case}) \qquad (15)$$

where $-7.5 \le V_m \le 7.5$

The end of the outflow occurs when the flow of blood, $Q_b$, is less than $Q_{min}$. The proper choice of $Q_{min}$ causes the membranes to empty properly and allows the controller to adapt to different membrane fill volumes.

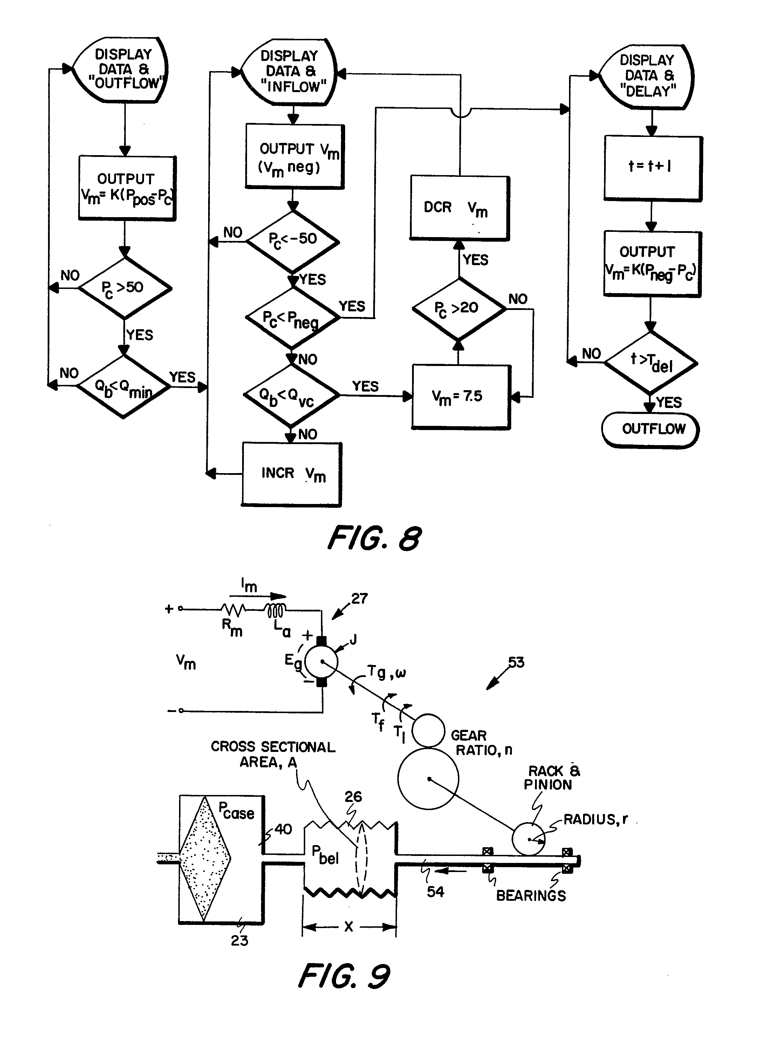

A general flow chart of the control algorith is shown in FIGURE 8, for control of the system as shown in FIGURE 7 (which will be referred to hereinafter as a pressure feedback controller). The limits of ± 50 mm Hg on pressure are needed to assure that a sufficient flow may reasonably be expected and thus the lack of flow is either venous collapse or the end of the outflow.

The control system of this invention may also utilize motor current ($I_m$) to replace the outflow from the pressure transducer. A pressure feedback system has an inherent problem in that it controls a monitored signal. If, for example, the pressure transducer amplifier drifts with time, the system could indicate the proper pressure limits even though the actual pressures are offset. Also, if motor current is not limited, it is possible that the motor could be overdriven during build up to the desired pressure. In addition, elimination of the pressure transducer and its associated amplifier simplies the system, at least for some apparatus.

The current signal is measured by using a small series resistor and operational amplifiers, and hence is easily implemented using amplifier unit 51 as brought out hereinabove.

In order to utilize motor current, the relationship between case pressure, motor current, and motor voltage must be derived. FIGURE 9 illustrates a schematic of the entire electrical and mechanical components of the pumping system. The relation between motor current and voltage is given by:

$$V_m = L_a \dot{I}_m + R_m I_m + E_g \qquad (16)$$

where $L_a$ = armature inductance

$I_m$ = motor current

$R_m$ = motor resistance

$E_g$ = Back EMF generated by motor

The value of $E_g$ is directly proportional to the motor velocity, $\omega$, that is:

$$E_g = K_e \omega \qquad (17)$$

where $K_e$ = voltage constant of motor

Combining equations (16) and (17) yields the basic electric equation of the motor:

$$V_m = L_a \dot{I}_m + R_m I_m + K_e \omega \qquad (18)$$

Denoting the mement of inertia of the motor by $J_m$ and the load by $J_1$, and letting $T_g$ denote the torque generated by the motor, one can sum the torques on the shaft to get:

$$T_g - T_1 - T_f = (J_m + J_1)\dot{\omega} \qquad (19)$$

where $T_1$ = load torque

$T_f$ = frictional torque

Since the magnetic field in the motor is constant, the current produces a proportional torque, or

$$T_g = K_t I_m \qquad (20)$$

where $K_t$ = motor torque constant

Equation (19) is the dynamic equation of the motor, and along with equations (17) and (18), the relation between the electrical and mechanical variables is described. The transfer function from motor voltage to angular velocity can be derived from the above equations, and is given by

$$\frac{\omega(s)}{V_m(s)} = \frac{(1/K_e)}{(s\tau_m+1)(s\tau_e+1)} \qquad (21)$$

where $\tau_m$ = mechanical time constant

$\tau_e$ = electrical time constant

And the values of the time constants above are given by:

$$\tau_m = (R_m J)/(K_e K_t) \qquad (22)$$

$$\tau_e = L_a/R_m \qquad (23)$$

This derivation assumes that the following holds

$$L_a \ll (R_m^2 J)/(K_e K_t) \qquad (24)$$

Equation (21) indicates that a step in motor voltage does not cause an instantaneous change in motor speed. The relation between motor current and voltage is given by:

$$K_t I_m = (J/K_e)(DV_m - L_a D^2 I_m - R_m D I_m) + T \qquad (25)$$

The transfer function from motor voltage to motor current is:

$$\frac{I_m(s)}{V_m(s)} = \frac{\tau_v s}{(s\tau_m + 1)(s\tau_e + 1)} \qquad (26)$$

where $\tau_v$ = voltage derivative gain

The value of this gain is given by:

$$\tau_v = J/(K_e K_t) \qquad (27)$$

Thus for a constant load torque, T, a step change in motor voltage will cause a spike in motor current. This phenomenon can be explained as follows. In steady state, a change in motor voltage causes a proportional change in motor speed. But to get a step change in motor velocity, an infinite torque at that instant of change is needed, thus the spike in current.

Steady state analysis is needed to relate the case pressure to the load torque, $T_1$. The torque is caused by two factors, the spring constant of the expandable bellows and the pressure in the bellows. The first term can be represented by:

$$T_{1s} = K_b nr\delta \qquad (28)$$

where $T_{ls}$ = load torque due to spring constant

$K_b$ = spring constant of bellows

n = gear ratio from motor to pinion

r = radius of the pinion gear

$\delta$ = displacement of bellows from equilibrium

The second term, representing the effect of bellows pressure, is given by:

$$T_{lp} = P_{bel}nrA \qquad (29)$$

where $T_{lp}$ = load torque due to bellows pressure

$P_{bel}$ = average pressure in bellows

A = cross sectional area of bellows

It is possible to relate to case pressure, $P_{case}$, to the bellows pressure by assuming a constant fluid resistance caused by the connecting tube, that is

$$P_{bel} = P_{case} + R_tQ_s \qquad (30)$$

Assuming laminar flow, and given the large tubing dimensions of the system, it can be concluded that the case pressure is closely approximated by the bellows pressure.

There are numerous components of the frictional torque, $T_f$. It is assumed that since the angular velocity is relatively small all viscous damping is negligible. Two sources of frictional torque have been examined, the bearing friction and worm gear friction.

The bearing friction is due to the load on the rack bearings caused by the weight of fluid in the bellows. Given the density of the fluid in the bellows and the coefficient of friction of the bearings, an estimate of the frictional torque is:

$$T_{fb} = 1.8 \times 10^{-6} x \ (23.5 - 2x), \text{ lbf in} \quad (31)$$

where X = expansion of bellows (in inches)

Thus, for the typical range from 5 to 9 inches, the maximum value of this torque is about $1 \times 10^{-4}$ inlbr. This is equivalent to the torque caused by 3 mm Hg, so is, for all intensive purposes, negligible.

The worm gear (of gear train 53) efficiency was estimated to be sixty percent (60%). Thus, the torque $T_{fw}$ is proportional to $T_1$, and given as:

$$T_{fw} = 0.67T_1 \quad (32)$$

Now, for this particular pumping system, Table 1 contains the known quantities in the previous equations.

<u>TABLE 1</u>

<u>DC Motor Specifications</u>

| | |
|---|---|
| Max. Nom. Voltage | 7.5 Volts |
| No-Load Speed | 7500 RPM |
| Back EMF Constant | 1.0 V/10 RPM |
| Max. Output | 2.4 Watts |
| Rotor Resistance | 5.7 Ohms |
| Torque Constant | 1.35 Oz-in/A |
| Moment of Inertia | 3.46 Kgm x $10^{-7}$ |
| Mechanical Time Constant | 21.0 msec |
| Average No-Load Current | 7.0 ma |
| Stall Torque | 1.76 Oz-in |

The electrical time constant was determined by clamping off the motor shaft and running a voltage step test. The step response of motor current indicated that $\tau_e$ was 0.07 msec and thus the motor inductance, $L_m$, is 0.4 mH. Plugging in the known values into equation (27) shows that $\tau_v$ to be 3.8 msec/$\Omega$. These values indicate that the motor current does spike for step changes in voltage, but the current very rapidly reaches a steady state level.

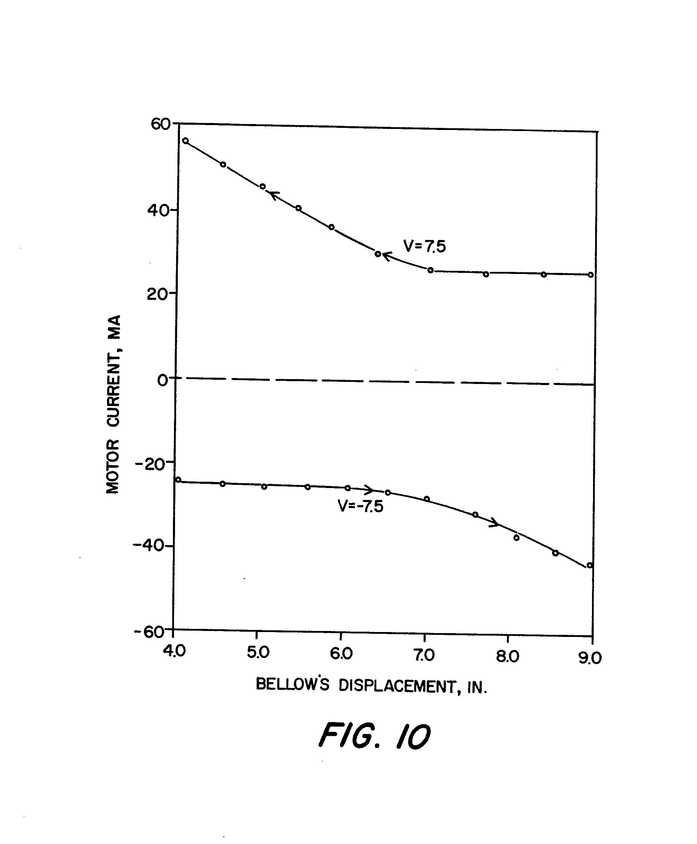

To experimentally evaluate the combined effect of the three terms, namely $T_{fb}$, $T_{fw}$, and $T_{ls}$, fluid was cycled into and out of a beaker. The motor current was recorded along with the position of the bellows. The results are shown in FIGURE 10 as an experimental plot of motor current versus bellows position for both inflow ($V_m = -7.5$) and outflow ($V_m = 7.5$). It can be readily seen that the spring effect, $T_{ls}$, is considerable. The sensitivity of the current-pressure relationship is given by:

$$I_m = (Anr/K_t)P_{case} \qquad (33)$$

$$= 0.286 \ (ma/mm \ Hg) \ P_{case}$$

Including the worm gear efficiency, this causes the factor to be 0.47 ma/mm Hg. Thus the measured current, as shown in FIGURE 10, is divided by 0.47 to obtain the equivalent pressures. For instance, 60 ma corresponds to about the same level as 125 mm Hg. Thus, the indication is that a softer bellows would enable much easier isolation of the pressure load term.

This analysis was used to develop a software program to control the bellows pump with motor current as the feedback. The function of the program is to duplicate the performance of the pressure controller without the use of a pressure measurement. The user inputs the same values to the system, that is, pressure ranges, delay time, and flow-reversal limits. Based on the theoretical analysis and experimental data of the previous section, the motor current is used as feedback to indicate case pressure.

The simple feedback algorithm used for the pressure controller cannot be used as the current controller program. Since a step change in voltage causes a spike in motor current, even if a steady state current level, $I_{pos}$, corresponding to the positive pressure, $P_{pos}$, is used, a linear feedback controller using $I_{pos}$ as desired value would not work. That is

$$V_m = K_g (I_{pos} - I_m) \qquad (34)$$

$$-7.5 < V_m < 7.5$$

would cause the motor voltage to oscillate between $\pm 7.50$ volts for sampling rates greater than the rate of decay of current spike. The current signal by itself has a very fast response, but it was corrupted with noise. An RC filter was used to average the signal but the decay was too slow, after the current spike, to allow for a linear feedback.

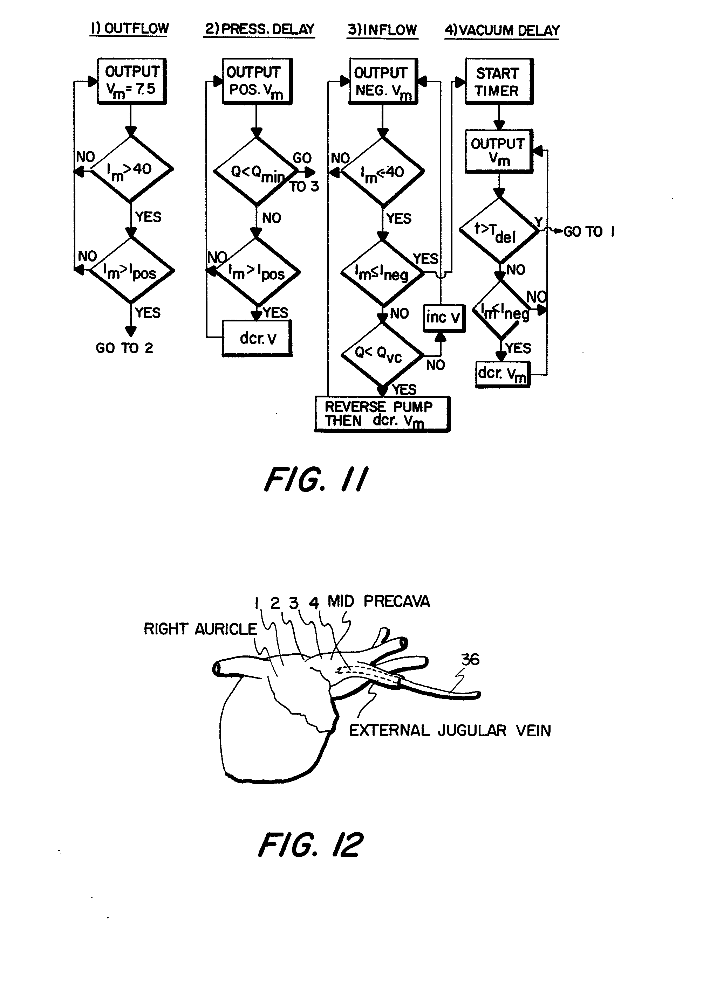

Again, the cycle was divided into different phases, namely, inflow, vacuum delay, outflow, and pressure delay. FIGURE 11 is a flowchart of the program.  Experimentally, the relationship of current to pressure was established so that $I_{pos}$ and $I_{neg}$ is determined from $P_{pos}$ and $P_{neg}$ for $V_m = \pm 7.50$ volts. During inflow, the motor voltage is set in the same manner as in the pressure controller.  The end of inflow occurs when the measured motor current exceeds $I_{neg}$.  When this occurs, the vacuum delay stage is entered.  It was found that, due to the worm gear drive, the motor can be stopped at this point and the vacuum can be held.  After the delay time interval, the motor voltage is set at +7.50 volts for outflow.  This phase continues until the motor current exceeds $I_{pos}$, then pressure delay begins.  Unlike the vacuum delay, the pressure delay phase requires additional bellows contraction to maintain the positive pressure.  A "creep" strategy is used to maintain the pressure, that is, the positive motor voltage is reduced if the current exceeded $I_{pos}$.  The pump gradually finds a level of slurry flow that holds the desired pressure. During this phase the flow is monitored, and if less than $Q_{min}$, then inflow begins.

An in vivo test was performed with a venous catheter as access.  A silicone rubber tube, 2.6 mm ID or 10 guage, was inserted into the jugular vein of an anesthesized dog.  There were two main goals of this experiment:  to evaluate the pumping system and to determine a good location of the catheter for dialysis. The test setup was similar to that shown in FIGURE 7 except that a simplified cell was used consisting of two smaller bellows connected in parallel in a case (such a cell exhibited the same response as the standard cell and was easier to work with), and the outflows of the flowmeter and pressure transducer were recorded on a two channel recorder.

The inputs to the controller are listed in Table 2.

### TABLE 2

#### Typical Pump Inputs

| | |
|---|---|
| Negative Pressure | -200 mm Hg |
| Positive Pressure | 100 mm Hg |
| Delay Time | 25 secs |
| Flow Limit | 80 ml/min |
| Vein Collapse Ind. | 25 ml/min |

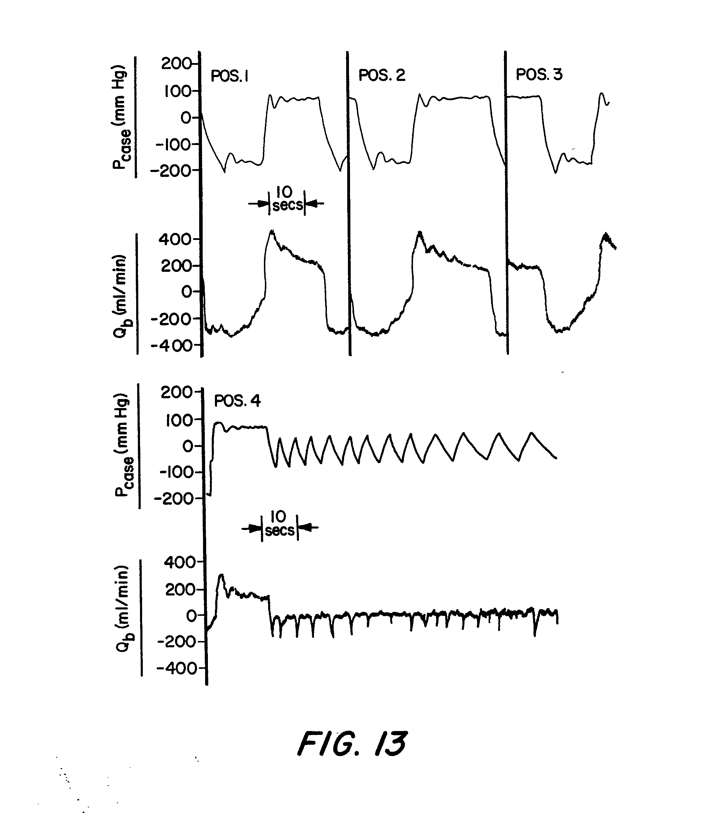

At the start of the test, the catheter was positioned next to the right atrium, called position 1 as shown in FIGURE 12. Additional positions (as shown in FIGURE 12) were tested and recorded by measuring the external tubing length. The responses of $P_{case}$ and $Q_b$ versus catheter location are plotted in FIGURE 13. The point three-quarters the distance between points 3 and 4, as shown in FIGURE 12, represented the best location as it is biologically compatible while still producing the desired flow. At point 4, as shown in FIGURE 12, the system tried to withdraw the blood slow enough to prevent collapse, but never could. The negative pressure jumps when the delay stage starts because of the elevation of the dog (which was approximately 3 feet), causing a positive pressure head.

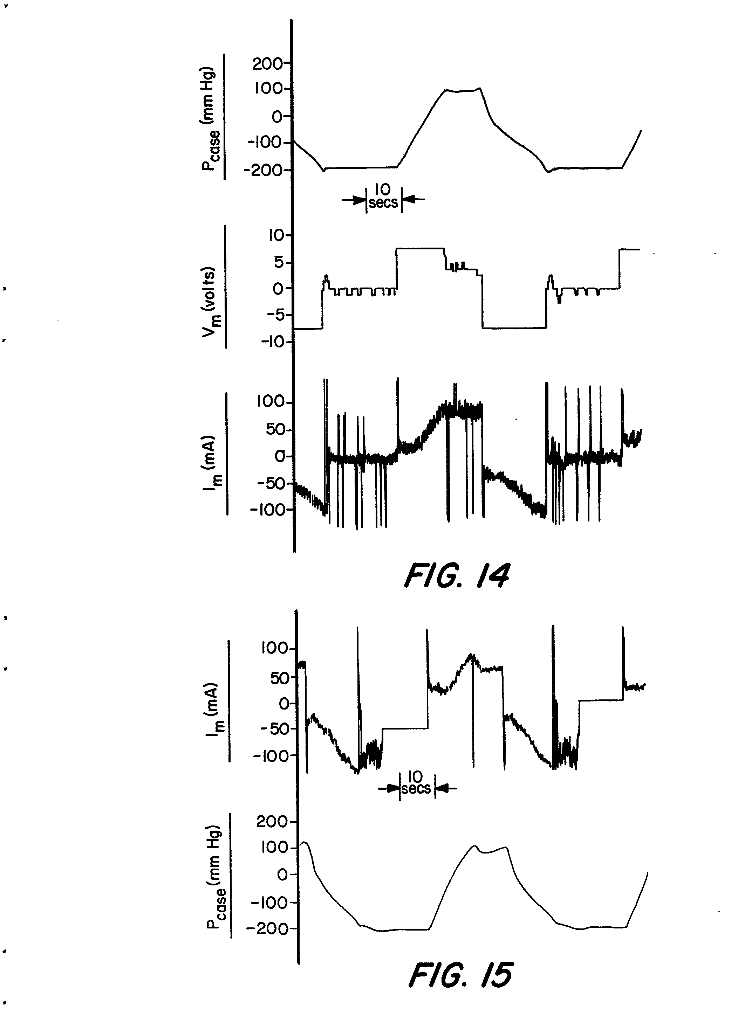

During the in vivo tests with the pressure controller, the motor current was also recorded. It was found that an RC filter was required to eliminate some of the signal noise. FIGURE 14 is a plot of motor current, pressure, and motor voltage. Note that, as predicted earlier, a step change in voltage caused a spike in motor current in the direction of that change. This phenomenon cannot be eliminated by any mechanical modification as it also occurs with just the motor by itself.

All testing with the current controller was performed in vitro with a beaker of water as the source of fluid. The water was used on both the blood and slurry side. A powder (such as Cellulosepuluer MN300, Brickman Instr., Westbury, New York) was used in the blood side water so that the flowmeter could detect the flow velocities. The values in Table 2 were used as input parameters. The system response is shown in FIGURE 15. It should be noted that the pressure waveform closely parallels the pressure controller response. There is a slight valley in the positive pressure delay due to the algorithm reducing the voltage too fast. But for safety reasons this is a better method of operation. Also, the current spikes still occur, but are in opposite direction of the current limits.

To accommodate the added bellows volume caused by ultrafiltration, a microswitch circuit (switches 57 and 58 as shown in FIGURE 7) was utilized to sense when bellows 26 overexpanded. When the bellows fills up with about one-half liter of ultrafiltrate, the bellows trips a switch at the end of the inflow stage. When this happens, a message can be printed on a visual display (such as a CRT) that tells the operator
to turn a valve so the excess fluid can be pumped out
of the bellows.  This type of arrangement permits the
pump system to ultrafiltrate any amount of volume using
a small bellows.

The visual display can also be utilized to
display case pressure, $P_{case}$, flow rate, $Q_b$, and motor
voltage, $V_m$.  Motor current can also be displayed to
make sure that the motor is not being overdriven.

A rotary potentiometer mounted on the pinion
shaft gives a voltage proportional to the position of
the bellows.  The pumping blood volumes can then be
indirectly determined.  To accomplish this, the case
compliance, $C_{case}$, defined earlier, plus the compliance
of the bellows itself and that associated with the case
air is determined by clamping off the blood line and
recording how much slurry volume is pumped to get +100
to -200 mm Hg case pressure.  Assuming a linear compliance, sufficient data is available to calculate the
fill volume pumped during each cycle.

During normal operation, the extreme bellows
positions are noted during each cycle, and the pumped
volume is given by:

$$Vol = A(x_{max} - s_{min}) - A(\Delta x) \qquad (35)$$

where A = bellows cross-sectional area

An accumulated total is displayed from these
volumes that gives an indication of treatment rate, as
the total session time is output.  As another aid to
the user, the inflow and outflow times are also shown (the inflow time in this case is defined to be the time of the delay and inflow phases combined).

During in vivo pump tests, chemical tests were also being conducted that required slight modifications of the system software. Blood samples were taken every half hour at two stages in the cycle. The inflow sample of about 20 cc, which represents the current condition of the patient's blood before the cycle treatment, was taken from the shunt directly just as inflow begins. An outflow sample was taken after that blood was treated by clamping off the tube from the shunt to dialyzer and drawing off all the blood in the cell into a syringe. This procedure confused the control unit, making it think the outflow cycle was over. The control unit then caused inflow to begin and, because the tube was clamped off, the controller believed the vein had collapsed. To alleviate this problem, a separate sub-routine was written that would be entered when taking samples. The inflow sample presented no problem, but when a user wishes to take an outflow sample, the user would push a key on the terminal at any time in the pumping cycle. The control unit then operates in its normal manner until the next delay phase is reached. When the system is five seconds from switching to outflow, a bell is caused to ring at the terminal telling the user that it is time to clamp off the blood line and get a syringe ready to withdraw the blood. When outflow begins, a second bell rings and the pump generates a case pressure of value $P_{pos}$. This in turn will pump all the blood out of the cell. The difference between this procedure and normal outflow is that the control unit does not check the flow rate and thus does not misinterpret the situation.

The system shown in FIGURE 7 utilizes a complete microcomputer system as the digital controller. In order to start the pump, it is necessary for the operator to insert a magnetic disc (such as a 5" magnetic disc), with the controller software, into a disc drive and then transfer the program from the disc to a random access memory (RAM). Next the user types in the required inputs; negative and positive pressure limits, time delay, and flow limit. Then the program execution can begin. This setup procedure is complicated and, while care must be exercised, it is useful in many applications.

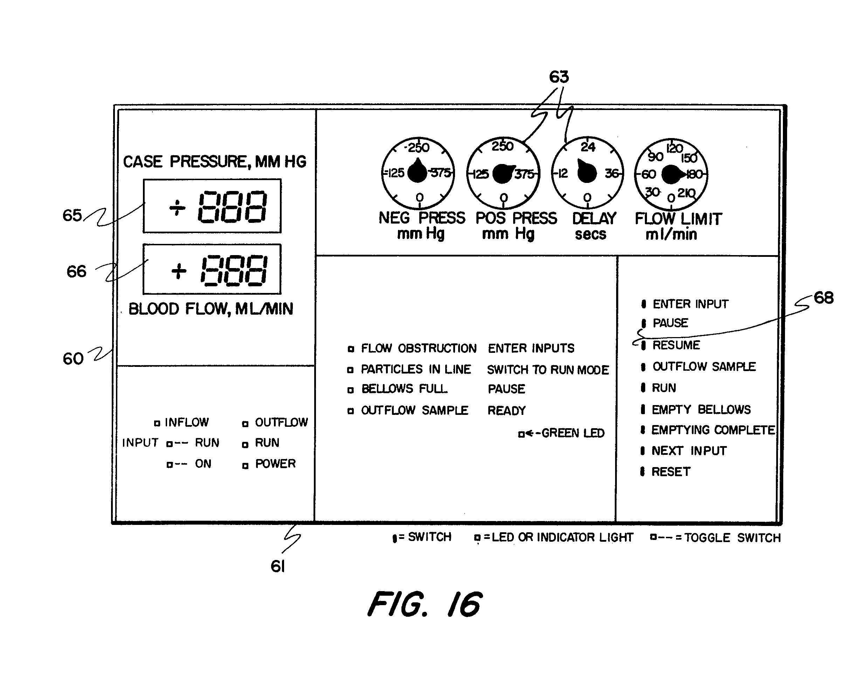

An alternate embodiment of the control system with operational and parameter selector at a front panel is shown in FIGURE 16. The control system shown closely resembles most instruments with easy patient operations and is therefore preferred for ordinary patient care. In addition, the system has software in permanent storage, and the amount of hardware required is minimized. The program for a pressure controller and current controller are written in the language BASIC and required a 16K interpreter program so that the total system memory requirements were 25K which were much too large to be stored in a read only memory (ROM).

There is, however, another BASIC available (Control Basic, Comemco, Mountain View, California) that is on two ROMs and spans just 4K of memory, so that the pressure controller and current controller can be combined into one program that required only 6K memory. This program can be permanently stored on three 2716 EPROMs (erasable programmable read only memories). A commercially available PC card (Single Card Computer, Cromemco) that is comprised of a Z80 microprocessor, four I/O ports, 400 bytes of random access memory (RAM), and sockets for 8K of ROM storage, was used for the controller which is shown in FIGURE 16. This 5" by 10" card, along with the A/D and D/A Card, when connected to appropriate power supplies, duplicates the performance of the disc-operated microcomputer system (shown in FIGURE 7) in every detail except the sampling rate is one-third as fast. A terminal for data input and output is still required. Table 3 lists the specifications for this system, including the components of the control option and the power consumed.

## TABLE 3

### Pumping System Specifications

| | | |
|---|---|---|
| 1. | PC Boards | 7 Channels A/D & D/A<br>Single Card Computer<br>EPROM Expander Board<br>I/O Display Interface Board |
| 2. | Memory | 400h RAM for stack<br>4K Control Basic<br>6K BTC-PC23 Controller |
| 3. | Power Requirements | 8 volts at +1.2A<br>+ 18 volts at 100MA<br>- 18 volts at 50MA<br>+ - volts |
| 4. | Transducers | Press. Trans. 1 v/200 mm Hg<br>Flowmeter 1 v/200 ml/min<br>I-V Conv. 1 v/50MA |
| 5. | Data Displayed | Case Press. Continuously<br>Blood Flow Continuously<br>Warning Conditions when<br>required |

In the embodiment shown in FIGURE 16, a self-contained control unit 60 is utilized which satisfies the conditions for the control system as set forth hereinabove and allows for user interrupts by providing push button switches. In addition, case pressure and flow rate are continuously displayed and warning conditions are indicated by lights and/or buzzers, all of which are located at the front panel 61 of controller unit 60.

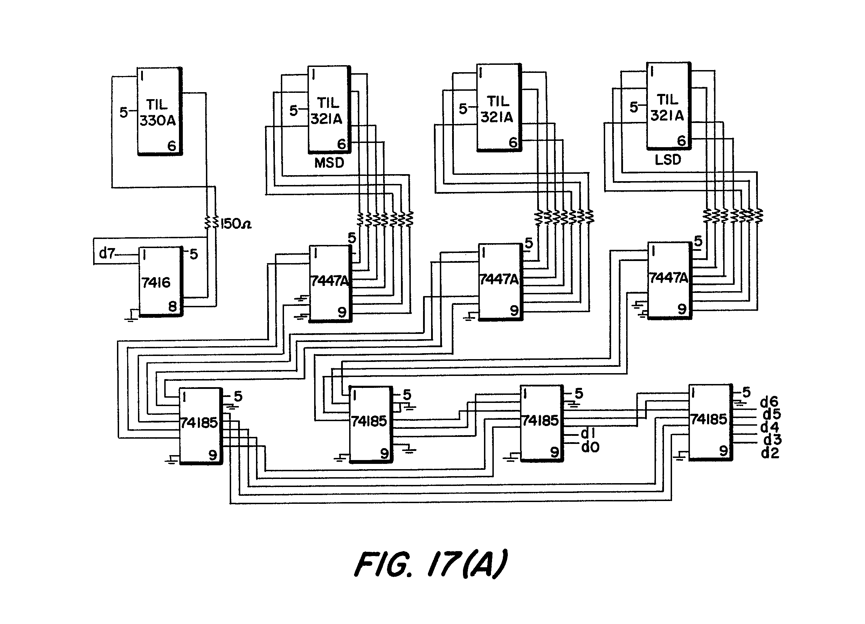

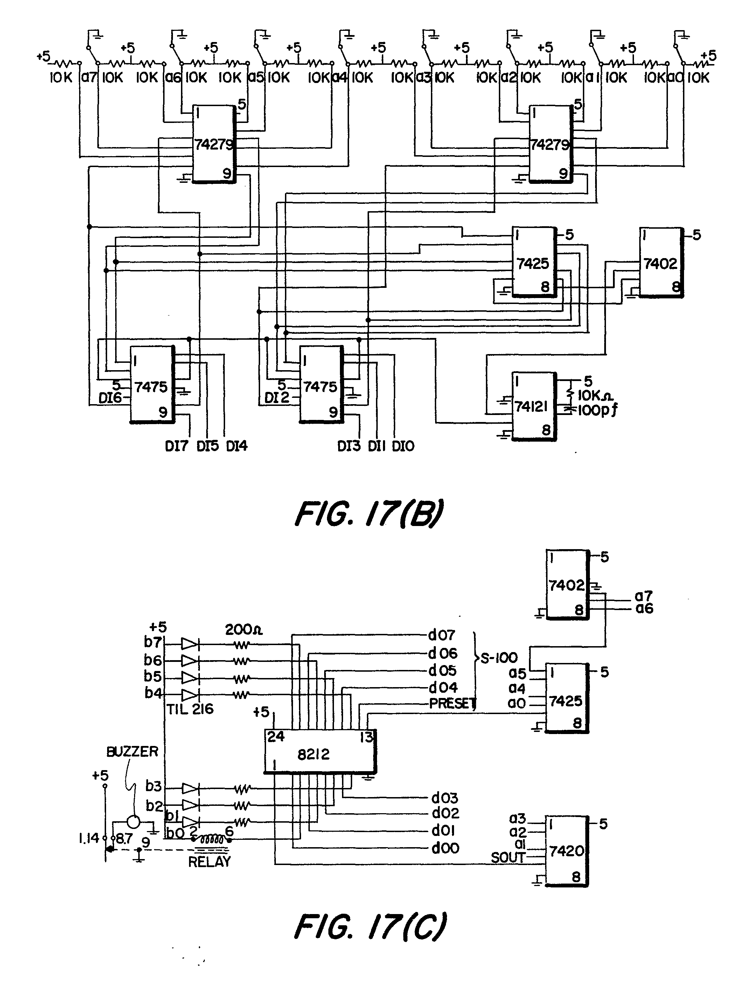

The user sets the input parameters by adjusting dials 63 in the top right corner of panel 61. Case pressure and blood flow rate are updated every second and displayed with three digit seven segment LEDs 65 and 66 at the upper left of panel 61. The user can interrupt normal operation by depressing the appropriate switch 68 on the botton right panel 61. For instance, if it is desired to stop the pump for a period of time, then the user can press PAUSE, and the RESUME when ready to continue. To accomplish the data I/O routine, three digital circuits are utilized that convert binary bit patterns to seven segment LED code (FIGURE 17 (A)), input binary patterns from eight switches (FIGURE 17 (B)), and output binary bit pattern to seven LEDs and a buzzer (FIGURE 17 (C)).

The program that controls the unit, a copy of which is attached hereto and is a part of this specification, also includes an analysis of the amplitude of the reflected ultrasound signal. This monitor has been demonstrated to have excellent sensitivity for either charcoal or air bubbles in the blood line. The existence of charcoal particles increases the amplitude and is detected by comparing the ratio of amplitudes during outflow stages. Air bubbles cause a spike in amplitude, but with the use of an RC filter, this signal can be drawn out long enough to be sensed. Thus, if the ratio of amplitude to a baseline amplitude ever exceeds a given amount, it can be concluded that air is in the line.

From a patient standpoint, control unit 60 has several advantages. Its operation involves mere adjustment of dials and pressing buttons, rather than operating a disc system on a computer. Also, if the values to be input have been specified, then there is no need to adjust the dials.

0052004

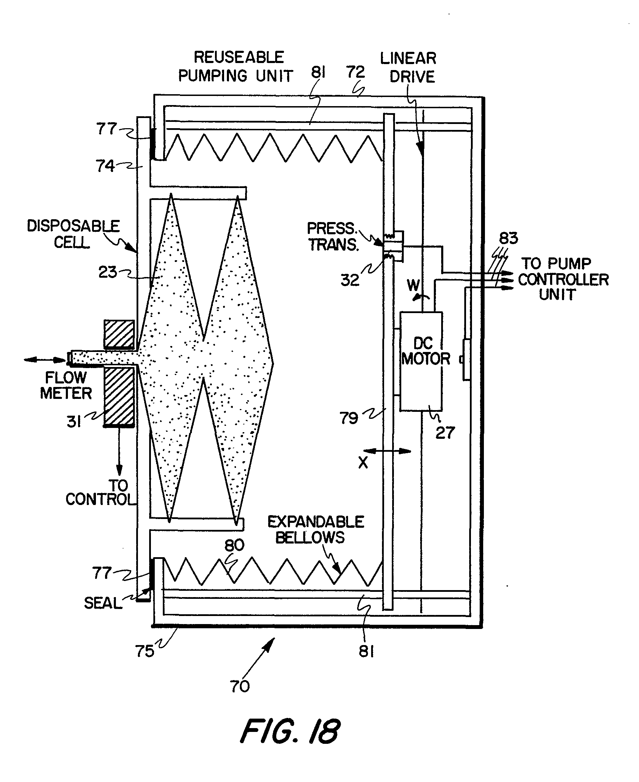

An alternate embodiment 70 of the bellows is shown in FIGURE 18. An expandable case 72 is utilized for generation of pressure. The treatment of cell 23 (consisting of a plurality of membrane packages) are preferably fabricated into a disposable unit 74, while the pumping unit 75 is reusable. The use of a large diameter bellows also allows for accommodation of more ultrafiltrate and requires less displacement during each cycle lessening the spring effect on current. Seal 77 (between the disposable unit and the pumping unit) can be accomplished by either a clamping system or a screw-type action. Pressure transducer 32 can be permanently affixed to the back plate 79 of bellows 80 with back plate 79 movable in longitudinal directions along guide shafts 81 fixed to the front and back walls of the pumping unit. If a limiting controller is used, DC motor 27, fixed to back plate 79 controls movement of the back plate through a linear drive arrangement connecting the motor with the guide shafts (as, for example, through a worm gear drive in engagement with threaded guide shafts), that is, $\dot{x}$ must be related to $\omega$ by a constant). The I/O could then be executed over one cable 83 to the pump controller unit.

A device utilizing ultrasound Doppler to monitor blood flowing in a vein and sensing the presence of contaminants therein is shown and claimed in U.S. Patent Application Serial No. 196,364, filed by Vernon L. Newhouse on October 14, 1980, and entitled "Method and Apparatus for Detecting Impurities in a Liquid Such as Blood", and assigned to the assignee of this invention.

This system can be used as flow sensor 31 to provide an output to control operation of the system to terminate operation if blood contamination is sensed. As brought out in this application, the flowing blood is monitored by an ultrasound Doppler system (which, if desired, can be simultaneously used for measuring the velocity of the blood flow) to detect contaminants in blood.

The ratio of the power reflected by blood cells and carbon particles can be predicted from knowledge of their relative numbers and the relative numbers and the relative differential cross section ($\sigma_d$). An applicable equation to calculate $\sigma_d$ is:

$$\sigma_d = \frac{1}{9} k^4 a^6 \left[ \frac{K_e - K}{K} + \frac{3p_e - 3p}{2p_e + p} \cos \theta \right]^2 \quad (36)$$

where

$K_e$ = adiabatic compressibility of the particle

$K$  = adiabatic compressibility of the medium

$P_e$ = density of the particle

$p$  = density of the medium

$\theta$  = scattering angle, or angle between incident beam and receiving beam (180° where the transmitting and receiving transducers are encased next to each other in the probe

$a$  = particle radius.

0052004

X-5647                              -47-

When the backscattered power is assumed to be equal to the sum of the powers backscattered by each particle, the power received by the transducer is:

$$P = \frac{nVk^4a^6I_iA}{9r^2} \left[ \frac{K_e-K}{K} + \frac{3p_e-3p}{2p_e+p} \cos \theta \right]^2 \quad (37)$$

where

n = number of particles per $cm^3$

V = volume of fluid observed

r = distance from transducer to this volume

$I_i$ = incident power

A = area of receiving aperture

k = $2\pi$/wavelength

It has been found that at a hemotocrit of 40, the backscattered power of blood is only about one fifth the power obtained by assuming independent scatters. A correction factor $A_d$ has therefore been introduced to account for this effect. This effect is known to occur for very heavy concentration of scatterers such as is provided by the erythrocytes in normal blood. For the much lower concentrations of carbon particles, no correction factor should be required. Thus, the corrected backscattered power may be written as $P_{erythrocytes} = P_e = A_dP$, where $A_d = 0.2$ for a hematocrit of 40.

From this information an equation can be derived for the ratio of power backscattered from red blood cells in a mixture of both, i.e.,

$$\frac{P_c}{P_e} = \frac{n_c}{A_c n_e} \left\{ \frac{a_c}{a_e} \right\}^6 \left[ \frac{\frac{K_c - K}{K} + \frac{3p_c - 3p}{2p_c + p}\cos\theta}{\frac{K_e - K}{K} + \frac{3p_e - 3p}{2p_e + p}\cos\theta} \right]^2 \quad (38)$$

In this equation, the subscripts c and e refer to carbon and erythrocytes, respectively. The values of the constants are shown in Table 1.

### TABLE 1

### Acoustic Properties of Blood and Carbon

|  | Density $(gm/cm^3)$ | Adiabatic Compressibility $(cm^2/dyne)$ | Concentration $(\#/cm^3)$ | Average Radius |
|---|---|---|---|---|
| Erythrocyte | 1.092 | $34.1 \times 10^{-12}$ | $5 \times 10^9$ (Ht = 40) | 2.75 |
| Carbon | 1.76 | $2.6 \times 10^{-12}$ |  |  |
| Plasma | 1.021 | $40.9 \times 10^{-12}$ |  |  |

In the presence of carbon particles of different radii, the term $a_c^6$ may be defined as:

$$a_c^6 = \sum a_{c_i}^6 / \sum n_{c_i} \quad (39)$$

where $n_{c_i}$ is the concentration of carbon particles of radius $a_{c_i}$.

0052004

To experimentally test for blood contaminant presence determination, movement of blood through a hemodialysis system was duplicated. The insonification of the blood was done while the blood was made to flow through a 3/8" ID silicone tube connecting two 500 ml Erlenmeyer flasks at different heights. The connecting tubing was submerged in a water bath, and each flask was equipped with a magnetic stirrer to keep the erythrocytes and carbon from settling.

A Parks, Inc. 10 MHz continuous directional Doppler flowmeter was initially used for measurement. Similar results were obtained with a 2.25 MHz pulsed Doppler system.

The transducer probe of the Doppler flowmeter contained both the transmitting and receiving crystals, and this probe was placed in the water bath about three cm from the silicone connecting tubing and at an angle of about 45° with respect to it.

Outdated human blood, warmed to about 30°C and filtered through a 60μ filter to remove large contaminants such as blood clots, was placed into the upper of the two Erlenmeyer flasks and allowed to flow through the tubing.

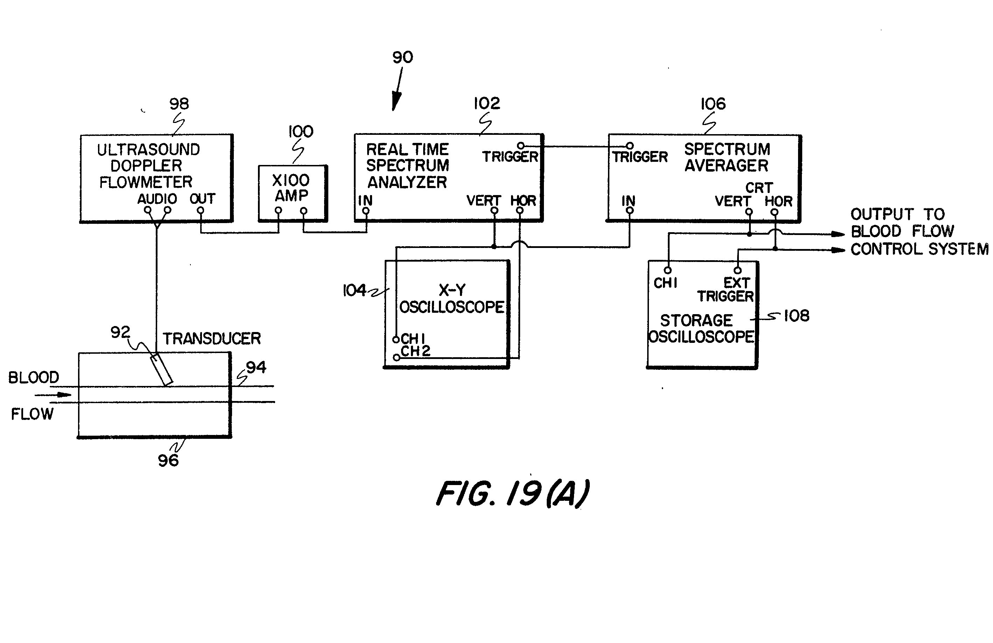

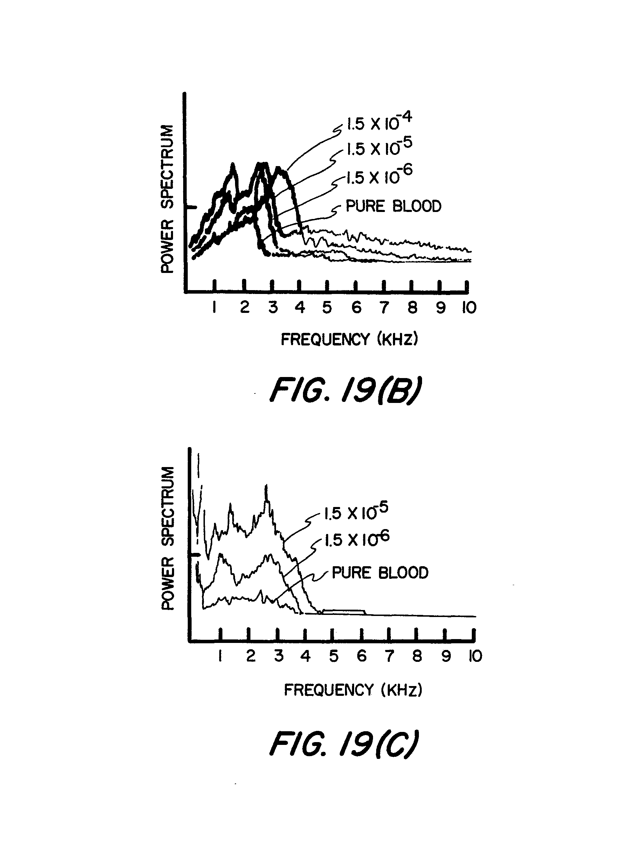

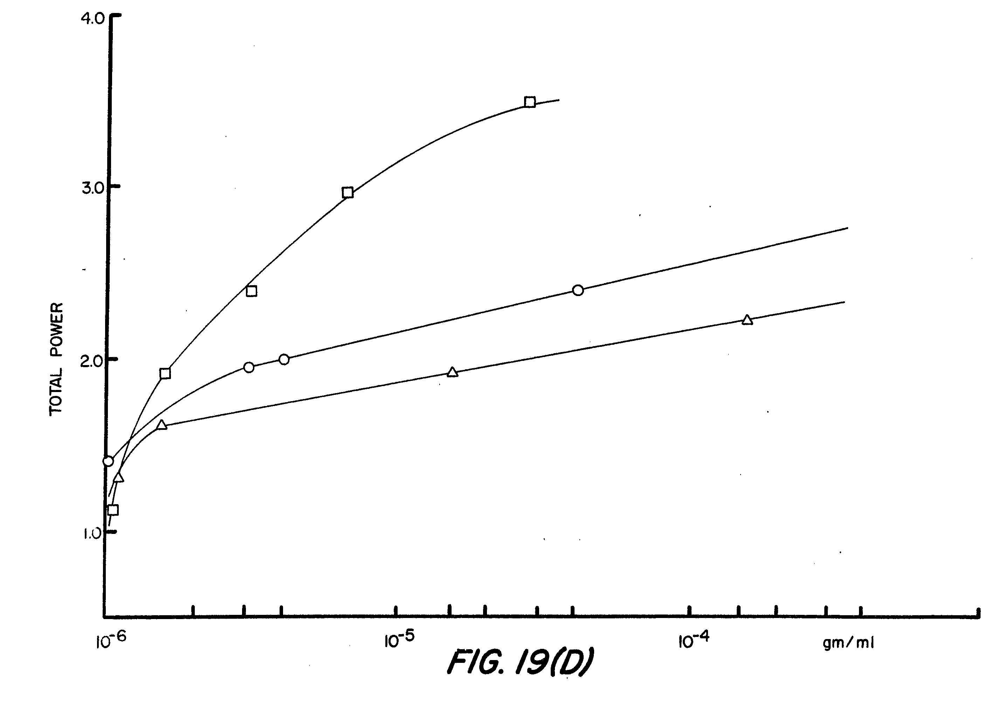

An electronic system 90 for monitoring the blood flow is shown in FIGURE 19(A). Ultrasonic Doppler radiation was directed from transducer 92 at an angle toward the blood flowing through vein 94. The reflected signal was received by transducer 92 and coupled therefrom to Doppler flowmeter 98 (either a Parks meter or pulsed Doppler system). The reflected signal was demodulated by the flowmeter and coupled to amplifier 100 where the signal indicative of reflected back-scattered power was amplified and then coupled to real time spectrum analyzer 102. Analyzer 102 received the data and converted it into a power spectrum every 50 ms.

The output of the spectrum analyzer was viewed by an oscilloscipe 104 and was also applied to spectrum averager 106. Spectrum averager 106 received a set number of spectra and then calculated their average. In this way, the randomness of the blood echo was minimized by averaging over a certain time period. The outlet was coupled to a second oscilloscope 108 to record the output of spectrum averager 106 and/or could be coupled to the blood flow control system for contamination and extermination and control when utilized in this invention.

For test purposes, a tube 94 was substituted for a vein and the blood caused to flow through tube 94 was first free of carbon particles and then later had different size carbon particles added thereto. The size distribution of the carbon particles used was estimated using a Coulter counter which uses screens to sift out particles of given sizes, and counts these particles.

Averages of 32 spectra were obtained. First, pure blood was allowed to flow through tubing 14 while a spectrum was obtained, then (when about 300 ml of blood was left in the upper flask) a small amount of carbon suspension was added. The spectrum of the scattering from the blood contaminated with carbon was then obtained. This procedure was repeated for different concentrations of carbon. For each different carbon concentration, the entire procedure required about two to three minutes, once the pure blood started to flow. It was felt that in this short time, the effect of the blood changing its properties due to exposure and handling would be minimal.

Similar experimental work has been carried out utilizing blood from an animal (dog) being dialyzed, which blood was routed through tubing in a water bath past a transducer in the same manner set forth above. This blood was analyzed with an electronic system like that shown in FIGURE 19(A) except that a power averaging circuit was used instead of a spectrum analyzer. This circuit provided an output proportional to the average back-scattered power, integrated over one second. The back-scattered echo of the pure blood of the dog was recorded over many dialysis cycles to get a baseline for future comparison. Then 60 ml of blood was withdrawn from the dog, and the dog was cut off from the dialyzer. This 60 ml of blood was allowed to go through several dialysis cycles (to make sure it was representative of pure blood), and then a known amount of carbon was added to it. This was repeated several times, with additional increments of carbon.

FIGURE 19(B) shows a Doppler spectrum (power vs. frequency) utilizing continuous Doppler for pure blood as well as a series of Doppler spectra obtained with increasing amounts of carbon ($1.5 \times 10^{-6}$ gm carbon/ml of blood; $1.5 \times 10^{-5}$ gm carbon/ml of blood;

and $1.5 \times 10^{-4}$ gm carbon/ml of blood). FIGURE 19(C) shows similar spectra using pulsed Doppler (pure blood; $1.5 \times 10^{-6}$ gm carbon/ml of blood; and $1.5 \times 10^{-5}$ gm carbon/ml of blood).

The data obtained from the Doppler spectra is shown graphed in FIGURE 19(D) as a function of carbon concentration. FIGURE 19(D) shows that the power detected by the receiving transducer increased with increasing carbon concentration, and the minimum amount of carbon detectable was found to be $1.5 \times 10^{-6}$ gm carbon per ml of blood. This was found to be true both in vivo and in vitro.

As can be appreciated from the foregoing, one constraint on the pump when utilizing a venous catheter is that the vein wall can obstruct blood flow. A flow rate monitor detects a "no flow" condition and the control unit attempts to prevent another occurrence. Extreme case pressures can be generated by a bellows type pump due to the limiting compliance of the membranes. For this reason, a pressure transducer is utilized.

The pump is an expandable bellows that cycles slurry into and out of the case. A control algorithm uses the pressure as the fedback variable and defines a cycle to consist of the phases - inflow, delay, and outflow. This type of system was tested both in vitro and in vivo and was shown to operate with a venous catheter as access.

The case pressure signal can also be derived from the motor current signal with correction factors derived from the setup. Another control algorithm used motor current as the feedback variable rather than case
pressure.  An alternating embodiment of the system
eliminates the need for a terminal, and makes operation
simpler.

The control system of this invention can be
used in numerous activities, among which would be
presently used dialysis treatment systems.  In particular, however, the control unit of this invention is
particularly useful for reciprocal dialyzers and makes
it possible to dialyze patients utilizing venous
catheterization and a simple conduit access thereto.

### BTC PUMP CONTROLLER SOFTWARE

1) A/D Port Assignments

| | | | Pin | Color |
|---|---|---|---|---|
| 31 | Pressure | (Neg. Press. Input) | B | Pink |
| 30 | Current | (Pos. Press. Input) | C | L. Brn |
| 29 | Flow | (Time Delay Input) | D | Orange |
| 28 | Rotary Pot | (Outflow Lim. Input) | E | Gray |
| 27 | Microswitches | | F | Green |
| 26 | Particle Monitor | | H | Blue |

2) D/A Port Assignments

| | | Pin | Color |
|---|---|---|---|
| 26 | Motor Voltage | 7 | Yellow |
| 30 | Microswitch Circuit | 3 | D. Brn |
| | Analog Ground | 10 | White |

3) I/O Port Assignments

| | |
|---|---|
| 10 | Pressure Led Display (J1), Note A7=1 is + |
| 11 | Flow Led Display (J2) |
| 4 | Panel Switches (J3) |
| 14 | Led + Buzzer Outputs |

4) Panel Switch Functions

    A7  (128) = Enter Input   (Pressure Mode)

    A6  ( 64) = Pause

    A5  ( 32) = Resume

    A4  ( 16) = Outflow Sample

    A3  (  8) = Run

    A2  (  4) = Empty Bellows

    Al  (  2) = Emptying complete  (Current Mode)

    A0  (  1) = Next Input

5) Indicator Light Functions

    B7  (128) = Flow Obstruction   (Enter Inputs)

    B6  ( 64) = Particles in Line  (Switch to Run Mode)

    B5  ( 32) = Bellows Full    (Pause)

    B4  ( 16) = Outflow Sample   (Ready)

    B3  (  8) = Green Led

    B2  (  4) = Inflow

    Bl  (  2) = Outflow

    B0  (  1) = Buzzer

6) Transducer Sensitivities

    A) Parks Flow Meter    1 volt/200 ml/min Outflow

    B) Pressure Transducer 1 volt/200 mm hg

    C) Curr. to Volt. Con. 1 volt/50 ma

    D) Rotary Pot.        1 volt/170 ml

    E) Particle Mon.

7) List of Variables

A  = Lower limit of case pressure, input
AO = Upper limit of case pressure, input
B  = Delay time, input
BO = Lower limit of infusion flow, input
C  = Inflow/outflow indicator (1=in, 0=out)
CO = Outflow sample indicator (1=out, SAM)
D  = Delay counter
DO = VM increment interval
E  = Number of cycles
F  = Bit pattern input from switches
FO = Number output to D/A CH1, motor voltage
G  = Input from A/D port 31
GO = Input from A/D port 30
H  = Input from A/D port 29
HO = Case pressure in mm Hg
I  = Blood flow rate in ml/min
IO = VM Decrement interval
J  = Stores previous inflow voltage
JO = Temporary storage of FO during delay with IM
K  = IM outflow indicator (K=1 implies IM>IMAX)
KO = Old value of IM, stored for digital filtering
M  = Positive pressure delay indicator (=1)
R  = Output bit pattern to leds and buzzer
RO = Masked off bit pattern check of input switches
X  = Press. input during reversal on vein coll.
Y  = Limit of Pos. Current, a fctn of AO
Z  = Indicator of press/curr control (Z=1 IM)
ZO = Limit of Neg. current, A fctn of A

## USER OPERATION PROCEDURES AND OPTIONS

1) Basic operation

   A) Turn on power switch

   B) Be sure run/input switch is in input mode

   C) Indicator leds=enter inputs

   D) +100 on display indicates user should input the first input, neg. pressure.  The user adjusts the dial until the desired value is displayed on the lower display.  When it is, then type <ENTER INPUT>.

   E) The value for neg. pressure will now be displayed on the upper display.

   F) Press <NEXT INPUT> and +200 will appear indicating that the second input will now be entered.

   G) Proceed as above until all four inputs have been entered.

   H) Now press either current or pressure control button. +100 indicates pressure, +200 indicates current

   I) Switch the run/input switch to run

   J) Press <RUN> button when ready to execute program.

2) User options

   A) Press <PAUSE> to temporarily shutdown system then press <RESUME> to continue

   B) Press <OUTFLOW SAMPLE> to take an outflow sample. The system will indicate the user request and a buzzer will be set off approximately 5 seconds before the outflow phase begins.  The case pressure will be held at the user specified maximum pressure limit until the user presses <RESUME>

   C) Press <RESET> button to start the whole procedure over

3) System outputs during operation

   A) <FLOW OBSTRUCTION> will be displayed when, for some reason, there is insufficient flow (possibly vein collapse).  The system will sound an alarm and will reverse in an attempt to open the access up.  If continuous recycling and alarms occur shut off the system.

B) <BELLOWS FULL> indicates that one of the microswitches was tripped. It is assumed this was caused by the overexpansion of the bellows. An alarm will be sounded until the operator presses the <EMPTY BELLOWS> button. Be sure that the tubing valve was switched to allow for removal of the excess fluid to another reservoir. When the bellows is empty press the <EMPTYING COMPLETE> key. Now reconnect the system for normal operation, and press the <RESUME> key.

C) <PARTICLES IN LINE> indicates either particles or air is in the line. Examine the setup, and if okay then press the <RESUME> key.

## CONTROL BASIC PROGRAM LISTING

```
 10 Autorun               :Automatic startup
 20 Out(26)=0             :Hold Bellows at its current
                                               position
 30 Out(14)=136           :B7=B3=1, enter inputs
    Neg. Press. Input

 40 out(10)=153           :+100 on press, display
 50 A=in(31)              :read pot. for neg. press.
 60 out(11)=A             :output neg. value to flow display
 70 F=in(4)               :read switches settings
 80 F=and(F,128)          :mask off A7
 90 If F=128 goto 110     :enter input pressed
100 goto 50               :reread neg. press. pot
110 out(10)=A             :output neg. press. to press
                                               display
120 out(11)=128           :+000 to flow display
123 out(14)=1             :set off buzzer
127 out(14)=136           :B7=B3=1,enter inputs
130 F=in(4)               :read switch settings
140 F=and(F,1)            :mask off A0, next input
150 If F=1 goto 170       :next input pressed
160 goto 130              :loop til A0 pressed
```

### Pos. Press. Input

```
170 out(10)=178        :+200 on press. display
180 A0=in(30)          :Read pot. for pos. press.
190 out(11)=A0+128     :output pos. press. to flow
                                            display
200 F=in(4)            :read switch settings
210 F=and(F,128)       :mask off A7
220 If F=128 goto 240  :enter input pressed
230 goto 180           :reread pos. press, pot
240 out(10)=A0+128     :output pos. press. to press
                                            display
250 out(11)=128        :+000 to flow display
253 out(14)=1          :set off buzzer
257 out(14)=136        :B7=B3=1, enter inputs
260 F=in(4)            :read switch setting
270 F=and(F,1)         :mask off A0, next input
280 If F=1 goto 300    :next input pressed
290 goto 260           :loop til next input pressed
```

### Time Delay Input

```
300 out(10)=203        :+300 on press. display
310 B=in(29)           :read pot. for time delay
320 B=B/10             :0-50 second delay range
330 out(11)=B+128      :output time delay to flow
                                            display
340 F=in(4)            :read switch settings
350 F=and(F,128)       :mask off A7
360 If F=128 goto380   :enter input pressed
370 goto 310           :reread time delay
380 out(10)=B+128      :output time delay to press
                                            display
390 out(11)=128        :+000 to flow display
```

Time Delay Input (continued)

```
393 out(14)=1              :set off buzzer
397 out(14)=136            :B7=B3=1, enter inputs
400 F=in(4)                :read switch settings
410 F=and(F,1)             :mask off A0, next input
420 If F=1 goto 440        :next input pressed
430 goto 400               :loop til next input pressed
```

Low Flow Limit Input

```
440 out(10)=228            :+400 on press. display
450 BO=in(28)              :read pot. for flow rate limit
460 BO=BO/2                :0-250 ml/min range
470 out(11)=BO+128         :output flow lim. to flow
                                              display
480 F=in(4)                :read switch settings
490 F=and(F,128)           :mask off A7
500 IF F=128 goto 520      :enter input pressed
510 goto 450               :reread flow limit
520 out(10)=BO+128         :output flow limit to press
                                              display
530 out(11)=128            :+000 to flow display
533 out(14)=1              :set off buzzer
537 out(14)=136            :B7=B3=1, enter inputs
540 F=in(4)                :read switch settings
550 F=and(F,1)             :mask off A0, next input
560 If F=1 goto 580        :next input pressed
570 goto 540               :loop til next input pressed
```

### Pressure or Current Control

```
580 Z=10                    :arbitarily set Z>3
581 F=in(4)                 :read switch settings
583 R=and(F, 128)           :mask off A7, pressure
586 IF R=128 Z=0            :pressure control
588 R=and(F,2)              :mask off A1, current
590 IF R=2 Z=1              :current control
592 IF Z>3 goto 581         :loop til mode selected
593 out(10)=153+Z*25        :+100 for P, +200 for IM
594 out(11)=128             :+000 to flow display
595 out(14)=1               :set off buzzer
600 out(14)=72              :B6=B3=1, switch to run
```

### Wait for startup command

```
610 F=in(4)                 :read switch settings
620 F=and(F,8)              :mask off A2
630 IF F=8 goto 650         :run pressed
640 goto 610                :loop til run pressed
```

### Adjust Input Variables

```
650 A=-4*A                  :0-125 to 0-500
660 A0=4*A0                 :0-125 to 0-500
670 B=4*K*B                 :0-12 to 0-, note K variable
680 BO=4*BO                 :0-62 to 0-248
```

### Initialize system

```
690 C=1                     :flow indicator to inflow
695 M=0                     :zero press delay ind for out sam
697 K=0                     :zero press delay indicator
700 CO=0                    :no outflow sample
710 D=0                     :zero delay counter
```

**0052004**

Initialize system (continued)

```
720 DO=0                    :zero increment interval
730 IO=0                    :zero decrement interval
740 E=0                     :zero number of cycles
750 FO=131                  :VM set to -7.50 volts
753 Y=FCTN(A0)              :calculate equivalent curr
                                              input
757 Z0=FCTN(A)              :calculate equivalent neg.
                                           curr input
```

Start of Loop

```
760 out(26)=F0             :output motor voltage
770 out(30)=127            :output voltage to uswitch
                                            circuit
780 IF in(27)<100 goto     :microswitch tripped
              1850
790 IF in(27)>130 goto     :microswitch tripped
              1860
800 G=in(31):if G>127      :input pressure
              G=G-256
805 KO=GO                  :store old value of motor
                                          current
810 GO=in(30)              :input motor current
820 IF GO>127 GO=GO-256    :25 comp to decimal
830 H=in(29)               :input flow rate
840 IF H>127 H=H-256       :25 comp to decimal
850 HO=4*G:I=4*H           :adjust inputs to mmHg and MA
```

Output Flow and Pressure

```
860 IF G>=0 G=G+128        :if pos. set MSB to 1
870 IF G<0 G=ABS(G)        :if neg. take ABS valve
880 out(10)=G              :output press. to display
890 If H>=0 H=H+128        :if pos. set MSB to 1
900 If H<0 H=ABS(H)        :if neg. take ABS value
910 out(11)=H              :output flow to display
```

Output LED Display

```
920 R=0                    :zero output bit pattern
930 If C=1 R=R+4           :B2=1 for inflow
940 If C=0 R=R+2           :B1=1 for outflow
950 If CO=1 R=R+16         :B4=1 for outflow sample
960 out(14)=R              :outputs to leds and buzzer
```

Input Switch Settings

```
970 F=in(4)               :read switch settings
980 RO=and(F,64)          :mask A6 (pause)
990 If RO=64 goto 1740     :user requested pause
1000 RO=and(F,16)         :mask A4 (outflow sample)
1010 If RO=16 CO=1        :outflow sample.
```

Filter Motor Current

```
1012 IF GO>100 GO=KO      :filter out pos. spikes
1017 IF GO<-100 GO=KO     :filter out neg. spikes
```

Determine Cycle Phase

```
1020 IF C=0 goto 1630     :if outflow goto press outflow
1030 IF D>0 goto 1320     :if delay goto press delay
1035 IF Z=1 goto 2030     :motor current control
```

Inflow with Press. Trans.

```
1040 IF HO<=(A+20) goto   :inflow to delay
           1260
1050 IF HO>(A/8) goto     :loop back til a vacuum
           760
1060 IF ABS(I)<25 goto    :vein collapse test
           1140
1070 IF DO=5 goto 1100    :VM Inc. interval check
1080 DO=DO+1              :increment the interval check
1090 goto 760            :loop back
1100 FO=FO-2             :VM=VM-0.04, increase
```

```
        Inflow with Press. Trans. (continued)

1110 IF FO<131 FO=131      :adjust VM>-7.50
1120 DO=0                  :zero inc. interval count
1130 goto 760              :loop back
1140 IF I0=2 goto 1170     :decrement interval check
1150 I0=I0+1               :inc. interval count
1160 goto 760              :loop back
1170 out(14)=129           :B7=B0=1, buzzer and flow obs.
1180 out(26)=100           :VM=+2.00 volts
1190 X=INP(31)             :input pressure
1200 IF X>127 X=X-256      :25 comp to decimal
1210 IF X<5 goto 1180      :loop til pcase=20 mm Hg
1220 FO=FO+2               :VM=VM+0.02, decrease
1230 I0=0                  :zero decr. interval
1240 IF FO>255 FO=255      :VM>=0.01 volts
1250 goto 760              :loop back
1260 D=0                   :zero delay counter
1270 J=FO                  :store VM for next cycle
1280 FO=A-HO               :feedback
1290 gosub 1800            :adjust to -7.5 to 7.5
1300 D=D+1                 :incr. delay count
1310 goto 760              :loop back

        Delay with Press. Trans.

1320 IF Z=1 goto 2090      :motor current control
1330 FO=A-HO               :feedback, gain=IV/50mmHg
1340 gosub 1800            :adjust to -7.5 to 7.5
1350 D=D+1                 :increment loop count
1360 IF D=B-5 goto 1380    :5 sec. before end of delay
1370 goto 1400             :skip bell output
1380 IF CO→1 goto 1400     :not in outflow sample
```

```
          Delay with Press. Trans. (continued)
     1390 out(14)=1              :set off buzzer
     1400 IF D>B goto 1420       :end of delay stage
     1410 goto 760               :loop back
     1420 IF CO=1 goto 1460      :outflow sample check
     1430 D=0                    :zero loop counter
     1440 C=0                    :outflow indicator zeroed
     1450 goto 760               :loop back

          Outflow Sample

     1460 out(26)=0              :temporarily shutdown motor
     1470 out(14)=1              :set off buzzer
     1480 out(14)=16             :set A4=1, outflow sample
     1490 F=in(4)                :read switch settings
     1500 F=and(F,32)            :mask off A5, resume
     1510 IF F=32 goto 1580      :resume key check
     1520 G=in(31)               :input from press. port
     1530 IF G>127 G=G-256       :2S comp to decimal
     1540 FO=(A0-(4*G))          :feedback
     1550 gosub 1800             :adjust to -7.5 to 7.5
     1560 out(26)=F0             :output VM
     1570 goto 1490              :loop til resume pressed
     1580 C=1                    :inflow indicator
     1590 E=E+1                  :increment cycle count
     1600 F0=J                   :VM from previous system
     1610 C0=0                   :zero outflow sample
     1620 goto 760               :loop back
```

### Outflow with Press. Trans.

```
1630 IF Z=1 goto 2160        :motor current controller
1640 F0=A0-H0                 :feedback, Gain=IV/50mmHg
1650 gosub 1800              :adjust to -7.5 to 7.5
1660 IF H0>(A0/2)goto        :IS pcase sufficiently pos.
          1680
1670 goto 760                :loop back
1680 if ABS(I)<B0 goto       :1S outflow cycle over
          1700
1690 goto 760                :loop back
1700 C=1                     :inflow begins
1710 F0=J                    :VM to previous cycle
1720 E=E+1                   :increment cycle count
1730 goto 760                :loop back
```

### Pause Subroutine

```
1740 out(26)=0               :stop bellows
1750 out(14)=40              :B5=B3=1, pause
1760 F=in(4)                 :read switch settings
1770 F=and(F,32)             :mask off A5, resume
1780 IF F=32 goto 760        :resume operation
1790 goto 1760               :loop til resume pressed
```

### Motor Voltage Adjustment Subroutine

```
1800 IF F0>125 F0=125        :VM<7.5
1810 IF F0<-125 F0=125       :VM>-7.5
1820 IF F0<0 then 1840       :dec. to 2S comp
1830 goto 1850               :pos. to no adjust needed
1840 F0=F0+256               :decimal to 2S comp
1850 return                  :return to call location
```

Bellows Emptying Subroutine

```
1860 out(26)=0              :stop bellows
1870 out(14)=33             :B5=B0=1, buzzer and bell full
1880 F=in(4)                :read switch settings
1890 F=and(F,4)             :mask off A2
1900 IF F=4 goto 1920       :was empty bellows pressed
1910 goto 1890              :loop til A2 pressed
1920 out(14)=32             :B5=1, bellows full
1925 out(26)=100            :VM=+2.00
1930 F=in(4)                :read switch settings
1940 F=and(F,2)             :mask off A1
1950 IF F=2 goto 1970       :was stop pressed
1960 goto 1925              :loop til bellows empty
1970 out(14)=24             :B4=B3=1, ready
1980 out(26)=0              :stop bellows
1990 F=in(4)                :read switch settings
2000 F=and(F,32)            :mask off A5
2010 IF F=32 goto 760       :resume operation
2020 goto 1990              :loop til resume pressed
```

Inflow with Motor Current

```
2030 F0=131                :VM=-7.50
2040 IF G0<Z0 goto 2084    :inflow to delay
2042 IF GO>(Z0/2) goto 760 :loop back til a vacuum
2044 IF ABS(I)<25 goto 2060 :vein collapse test
2046 IF D0=5 goto 2052     :VM inc. interval check
2048 D0=D0+1               :increment the interval check
2050 goto 760              :loop back
2052 F0=F0-2               :VM=VM-0.04, increase
2054 IF F0<131 F0=131      :adjust VM>=-7.50
```

0052004

### Inflow with Motor Current (continued)

```
2056 D0=0                :zero Inc. interval count
2058 goto 760            :loop back
2060 IF I0=2 goto 2066   :decrement interval check
2062 I0=I0+1             :inc. interval count
2064 goto 760            :loop back
2066 out(14)=129         :R7=B0=1, buzzer and flow obs.
2068 out(26)=100         :VM=+2.00
2070 X=INP(30)           :input motor current
2072 IF X>127 X=X-256    :2S comp to decimal
2074 IF X<(Z0/2) goto    :loop til pos. press.
2076 F0=F0+2        2068 :VM=VM+0.04, decrease
2078 I0=0                :zero decr. Interval
2080 IF F0>255 F0=255    :VM>=0.01
2082 goto 760            :loop back
2084 D=1                 :incr. delay count
2086 goto 760            :loop back to start delay
```

### Vacuum Delay with Motor Current

```
2090 F0=0               :VM=+0.00
2092 D=D+1              :incr. delay count
2094 IF D=B-5 goto 2098 :5 SEC before end of delay
2096 goto 2102          :skip bell output
2098 IF C0+1 goto 2101  :not in outflow sample
2100 out(14)=1          :set off buzzer
2102 IF D>B goto 2106   :end of delay stage
2104 goto 760           :loop back
2106 IF C0=1 goto 2114  :outflow sample check
2108 D=0                :zero loop counter
2110 C=0               :outflow indicator set
2112 goto 760           :loop back
```

Outflow Sample with Motor Current

```
2114 out(26)=0                 :temporarily shutdown motor
2116 out(14)=1                 :set off buzzer
2118 out(14)=16                :set A4=1, outflow sample
2120 F=in(4)                   :read switch settings
2122 F=and(F,32)               :mask off A5, resume
2124 IF F=32 goto 2138         :resume key check
2126 go=in(30)                 :input from motor curr.
2128 IF GO>127 GO=GO-256       :2S comp to decimal
2130 F0=125                    :VM=+7.50
2132 IF M=1 F0=0               :pos. press. delay
2134 out(26)=F0                :output VM
2136 goto 2120                 :loop til resume pressed
2138 C=1                       :inflow indicator
2139 M=0                       :zero press delay indicator
2140 E=E+1                     :increment cycle count
2142 C0=0                      :zero outflow sample
2144 goto 760                  :loop back
```

Outflow with Motor Current

```
2160 F0=125                    :VM=+7.50
2170 IF GO>Y K=1               :IS I>IMAX
2180 IF K>0 goto 2200          :press delay
2190 goto 760                  :loop back
```

Press, Delay with Motor Current

```
2200 F0=40                     :VM=+2.40
2210 IF ABS(I)<B0 goto         :IS outflow cycle over
          2290
2220 K=K+1                     :increment counter
2230 IF K>2 F0=J0              :digital filtering
2240 IF G0<(Y-10) goto         :prevent over-press
          2270
```

0052004

X-5647                          -69-

Press, Delay with Motor Current (continued)

```
2250 F0=F0-5              :VM=VM-0.1
2260 IF F0<0 F0=0         :VM>=0.00
2270 J0=F0                :store value
2280 goto 760             :loop back
2290 C=1                  :inflow set
2300 F0=131               :VM=-7.50
2310 E=E+1                :increment cycle count
2320 K=0                  :zero press. delay ind.
2330 goto 760             :loop back
```

CLAIMS

1.    A system for controlling biologic fluid flow, which comprises:

fluid flow effecting means for causing fluid flow in a predetermined path;

first monitoring means for monitoring fluid flow in the predetermined path and providing an output indicative of at least one preselected parameter thereof;

second monitoring means connected with the fluid flow effecting means and providing an output indicative of at least one preselected parameter thereof; and

processing means connected with the first and second monitoring means and the fluid flow effecting means to receive the outputs from the first and second monitoring means, and, at least partially responsive thereto, providing an output to the fluid flow effecting means to automatically control fluid flow in the predetermined path.

2.    The system of Claim 1 wherein the fluid flow effecting means includes pressure means for varying the pressure exerted on the fluid to cause the fluid flow, and pressure control means for controlling the pressure means with the pressure control means receiving the output from the processing means.

3.    The system of Claim 2 wherein the pressure control means includes a motor and motor control means connected to receive the output from the processing means.

4.    The control system of Claim 3 wherein the second monitoring means includes means for sensing motor current and providing an output indicative thereof to the processing means.

5. The system of Claim 2 wherein the pressure means includes a sealed case having the fluid in one portion thereof, and a pressure actuator connected with the sealed case to establish pressure on the fluid within the sealed case.

6. The system of Claim 5 wherein the second monitoring means includes pressure sensing means for sensing the pressure within the sealed case and providing an electrical output signal indicative thereof.

7. The system of Claim 5 wherein the sealed case has at least one deformable membrane, and wherein the pressure actuator introduces a fluid under pressure into the sealed case at the other side of the membrane to control deforming of the membrane.

8. The system of Claim 1 wherein at least one of the first and second monitoring means provides an output indicative of improper operation with respect to fluid flow and provides an output indicative thereof to the processing means, and wherein the processing means, responsive to receipt of the output indicative of improper operation, provides an output to the fluid flow effecting means to cause termination of fluid flow or correction of the improper operation.

9. A system for controlling biologic fluid flow in a reciprocating dialyzer apparatus wherein fluid flow into and out of the apparatus is through a single conduit, which comprises:

fluid flow effecting means for causing fluid flow in reciprocal directions through the single conduit of the reciprocating dialyzer apparatus;

monitoring means connected with the fluid flow effecting means and providing an output indicative of at least one preselected parameter thereof; and

processing means connected with the monitoring means and the fluid flow effecting means to receive the outputs therefrom and, at least partially responsive thereto, providing an output to the fluid flow effecting means to automatically control fluid flow through the single conduit in reciprocal directions in a predetermined pattern.

10. The system of Claim 9 wherein the fluid flow effecting means includes pressure means for varying the pressure exerted on the fluid to cause the fluid flow through the single conduit in the reciprocal directions, and pressure control means for controlling the pressure means with the pressure control means receiving the output from the processing means.

11. The system of Claim 10 wherein the monitoring means includes pressuring monitoring means for monitoring the pressure exerted on the fluid.

12. A system for controlling blood flow in a reciprocating dialyzer apparatus, which comprises:

means defining a blood chamber having a pressure responsive variable volume;

pressure means connected with the blood chamber defining means for establishing the volume of the blood chamber;

conduit means communicating with the blood chamber for blood flow therethrough to and from the blood chamber due to a change in volume of the blood chamber;

first and second monitoring means for monitoring the rate of flow through the conduit means and for monitoring pressures exerted by the pressure means, and providing electrical signal outputs indicative thereof;

programming means for receiving the electrical signal outputs from the monitoring means and establishing a program to effect cycling of blood flow through the conduit means to and from the blood chamber, the programming means providing a control signal output; and

control means receiving the control signal output from the programming means and responsive thereto controlling the pressure exerted by the pressure means.

13. The system of Claim 12 wherein the second monitoring means is connected with the control means to monitor motor current as an indication of pressures exerted by the pressure means.

14. The system of Claim 12 wherein the programming means, responsive to the electrical signal outputs from the first and second monitoring means indicative of an unsafe condition for operation of the dialyzer apparatus, causes termination of blood flow or correction of the unsafe condition.

15. A method for controlling biologic fluid flow, which comprises:

sensing the rate of flow of biologic fluid in a path;

sensing the amount of force necessary to produce fluid flow in the path;

determining optimum flow rates for the fluid in the path in view of the sensed rate and sensed amount of force necessary to produce the flow rate; and

controlling the flow rate to maintain flow at said optimum flow rate.

16.  The method of Claim 15 wherein the method includes determining unsafe operating conditions from at least one of the rate of flow and/or amount of force sensing methods, and responsive thereto, varying the method to effect termination or correction of the operation.

17.  A method for controlling blood flow in a reciprocating dialyzer apparatus, which comprises:

establishing a blood chamber having a pressure responsive volume;

providing a pressure source capable of controlling the volume of the blood chamber;

introducing blood into and withdrawing blood from the blood chamber through a single conduit;

monitoring the blood flow in the conduit;

monitoring the pressure exerted on the blood by the pressure source;

establishing a program for cycling the blood into and out of the blood chamber under the control of the pressure source;

modifying the program when the monitored blood flow and pressure are outside predetermined ranges; and

using the modified program to vary the pressure of the pressure source to effect cycling of the blood into and out of the blood chamber.

18.   The method of Claim 17 wherein the program is terminated if uncorrectable conditions causing unsafe operation are sensed by monitoring of the blood flow and pressure or when contaminants are sensed in the blood during monitoring of the blood flow.

FIG. 1

FIG. 2

FIG. 3

FIG. 4

0052004

FIG. 5

FIG. 6

**FIG. 7**

FIG. 8

FIG. 9

FIG. 10

0052004

FIG. II

FIG. 12

0052004

FIG. 13

0052004

FIG. 14

FIG. 15

CASE PRESSURE, MM HG

÷ 888

+ 888

BLOOD FLOW, ML/MIN

NEG PRESS mm Hg · POS PRESS mm Hg · DELAY secs · FLOW LIMIT ml/min

□ INFLOW  □ OUTFLOW
INPUT □-- RUN  □ RUN
□-- ON  □ POWER

□ FLOW OBSTRUCTION  ENTER INPUTS
□ PARTICLES IN LINE  SWITCH TO RUN MODE
□ BELLOWS FULL  PAUSE
□ OUTFLOW SAMPLE  READY
□◄ -GREEN LED

▮ ENTER INPUT
▮ PAUSE
▮ RESUME
▮ OUTFLOW SAMPLE
▮ RUN
▮ EMPTY BELLOWS
▮ EMPTYING COMPLETE
▮ NEXT INPUT
▮ RESET

▮ = SWITCH  □ = LED OR INDICATOR LIGHT  □-- = TOGGLE SWITCH

*FIG. 16*

FIG. 17(A)

*FIG. 17(B)*

*FIG. 17(C)*

FIG. 18

FIG. 19 (A)

FIG. 19(B)

FIG. 19(C)

FIG. 19(D)

0052004
Application number

### European Patent Office

## EUROPEAN SEARCH REPORT

EP 81 30 5311.3

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| X | US – A – 4 113 614 (ROLLO et al.)<br>* claims 1,2; column 2, lines 24 to 37; column 3, lines 16 to 42; column 7, lines 35 to 48 * | 1-3,<br>5-8,<br>15 | A 61 M 1/03 |
| Y | US – A – 3 946 731 (LICHTENSTEIN)<br>* claims 1,2,10,19,22; column 2, lines 16 to 58; column 3, lines 36 to 43; column 4, lines 23 to 35 * | 1-3,<br>5,7,8,<br>9-12,<br>14 | |
| Y | US – A – 4 153 554 (VON DER HEIDE et al.)<br>* claim 1; abstract; column 3, lines 17 to 23; column 7, lines 18 to 35; column 13, lines 8 to 14 * | 1-3,<br>5,7,8 | TECHNICAL FIELDS SEARCHED (Int.Cl.3)<br><br>A 61 M 1/03<br>B 01 D 31/00 |
| D,Y | US – A – 4 071 444 (ASH et al.)<br>* claims 1,2; column 2, lines 44 to 48 * | 9-12<br>14 | |
| Y | GB – A – 2 005 561 (BAXTER TRAVENOL LABORATORIES INC.)<br>* claims 1 to 3; page 3, lines 115 to 126 * | 1,8 | |
| A | FR – A1 – 2 368 963 (ABG – SEMCA)<br>* claims 1,6 * | 1-3 | |
| | ./.. | | |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| Berlin | 02-02-1982 | CLOT |

EPO Form 1503.1 06.78

**European Patent Office**

**EUROPEAN SEARCH REPORT**

0052004
Application number

EP 81 30 5311.3
- page 2 -

## DOCUMENTS CONSIDERED TO BE RELEVANT

| Category | Citation of document with indication, where appropriate, of relevant passages | Relevant to claim |
|---|---|---|
| A | DE - B - 1 566 633 (MILTON ROY CO.) <br> * claims 1,3 * | 1,8 |
| | -- | |
| P,X | WO - A1 - 80/02376 (LICHTENSTEIN) <br> * claims 1 to 5, 8; page 3, summary; <br> page 13, last paragraph * | 1-3, 5-8 |
| | ---- | |

**CLASSIFICATION OF THE APPLICATION (Int. Cl.³)**

**TECHNICAL FIELDS SEARCHED (Int. Cl.³)**